# EUROPEAN PATENT SPECIFICATION

(11) **EP 1 485 129 B1**
(45) Date of publication and mention of the grant of the patent: **10.01.2007**
(21) Application number: 03713601.7
(22) Date of filing: 21.02.2003
(51) Int. Cl.: A61K 39/395, B05D 3/00, B05D 3/04, C12Q 1/68, C12P 19/34, A61K 38/00, C07K 1/00

(54) **NANOSTRUCTURES CONTAINING PILIN PROTEIN**
NANOSTRUKTUREN MIT PILIN-PROTEIN
NANOSTRUCTURES CONTENANT UNE PROTEINE PILINE

(30) Priority: 21.02.2002 US 80608
(43) Date of publication of application: 15.12.2004
(73) Proprietor: Nanoframes, Inc., Boston, MA 02118 (US)
(72) Inventor: MAKOWSKI, Lee, Hinsdale, IL 60521 (US)
(74) Representative: Frei Patent Attorneys
(86) International application number: PCT/US2003/005340
(87) International publication number: WO 2003/072804

(56) References cited:
- WO-A-96/11947
- WO-A-02/059156
- WO-A2-00/68248
- US-A- 5 965 133
- US-A1- 2002 086 037
- MOLECULAR MICROBIOLOGY., vol. 37, no. 5, 2000, pages 1146-1158, XP002324494 GB BLACKWELL SCIENTIFIC, OXFORD.

### SEQUENCE LISTING

<110> Nanoframes, Inc.
   Makowski, Lee
<120> Nanostructures containing pilin proteins
<130> NANF.P-002WO
<140> PCT/US 03/05340
   <141> 2003-02-21
<150> 10/080,608
   <151> 2002-02-21
<160> 13
<170> PatentIn version 3.2
<210> 1
   <211> 18
   <212> PRT
   <213> Escherichia coli
<400> 1
   Ala Val Pro Gln Gly Gln Gly Lys Val Thr Phe Ser Gly Thr Val Val
   1               5                   10                  15
   Asp Ala
<210> 2
   <211> 53
   <212> PRT
   <213> Escherichia coli
<400> 2
   Pro Cys Gly Ile Asp Ala Ala Gln Ser Ala Asp Gln Ser Val Asp Phe
   1               5                   10                  15
   Gly Gln Ile Ser Lys Val Phe Leu Asp Asn Asp Gly Gln Thr Thr Pro
               20                  25                  30
   Lys Ala Phe Asp Ile Lys Leu Val Asn Cys Asp Ile Thr Asn Tyr Lys
           35                  40                  45
   Lys Pro Ala Thr Gly
       50
<210> 3
   <211> 33
   <212> PRT
   <213> Escherichia coli
<400> 3
   Met Ile Lys Ser Thr Gly Ala Leu Leu Leu Phe Ala Ala Leu Ser Ala
   1 5 10 15
   Gly Gln Ala Ile Ala Ser Asp Val Ala Phe Arg Gly Asn Leu Leu Asp
   20 25 30
   Arg
<210> 4
   <211> 37
   <212> PRT
   <213> Escherichia coli
<400> 4
   Pro Cys His Val Ser Gly Asp Ser Leu Asn Lys His Val Val Phe Lys
   1 5 10 15
   Thr Arg Ala Ser Arg Asp Phe Trp Tyr Pro Pro Gly Arg Ser Pro Thr
   20 25 30
   Glu Ser Phe Val Ile
   35
<210> 5
   <211> 55
   <212> PRT
   <213> Escherichia coli
<400> 5
   Met Arg Leu Arg Phe Ser Val Pro Leu Phe Phe Phe Gly Cys Val Phe
   1 5 10 15
   Val His Gly Val Phe Ala Gly Pro Phe Pro Pro Pro Gly Met Ser Leu
   20 25 30
   Pro Glu Tyr Trp Gly Glu Glu His Val Trp Trp Asp Gly Arg Ala Ala
   35 40 45
   Phe His Gly Glu Val Val Arg
   50 55
<210> 6
   <211> 15
   <212> PRT
   <213> Escherichia coli
<400> 6
   Pro Ala Cys Thr Leu Ala Met Glu Asp Ala Trp Gln Ile Ile Asp
   1               5                   10                  15
<210> 7
   <211> 36
   <212> PRT
   <213> Escherichia coli
<400> 7
   Met Lys Lys Ile Arg Gly Leu Cys Leu Pro Val Met Leu Gly Ala Val
   1               5                   10                  15
   Leu Met Ser Gln His Val His Ala Ala Asp Asn Leu Thr Phe Lys Gly
               20                  25                  30
   Lys Leu Ile Ile
           35
<210> 8
   <211> 34
   <212> PRT
   <213> Escherichia coli
<400> 8
   Pro Ala Cys Thr Val Thr Lys Ala Glu Val Asp Trp Gly Asn Val Glu
   1               5                   10                  15
   Ile Gln Thr Leu Ser Pro Asp Gly Ser Arg His Gln Lys Asp Phe Ser
               20                  25                  30
   Val Gly
<210> 9
   <211> 31
   <212> PRT
   <213> Escherichia coli
<400> 9
   Met Ala Arg Leu Ser Leu Phe Ile Ser Leu Leu Leu Thr Ser Val Ala
   1 5 10 15
   Val Leu Ala Asp Val Gln Ile Asn Ile Arg Gly Asn Val Tyr Ile
   20 25 30
<210> 10
   <211> 39
   <212> PRT
   <213> Escherichia coli
<400> 10
   Pro Pro Cys Thr Ile Asn Asn Gly Gln Asn Ile Val Val Asp Phe Gly
   1 5 10 15
   Asn Ile Asn Pro Glu His Val Asp Asn Ser Arg Gly Glu Ile Thr Lys
   20 25 30
   Thr Ile Ser Ile Ser Cys Thr
   35
<210> 11
   <211> 16
   <212> PRT
   <213> artificial
<220>
   <223> PapH-papK hybrid with added epitope: DNA coding for a Ras e pitope
<400> 11
   Glu Glu Tyr Ser Ala Met Arg Asp Gln Tyr Met Arg Thr Gly Glu Leu
   1 5 10 15
<210> 12
   <211> 6
   <212> PRT
   <213> Escherichia coli
<400> 12
   Asn Lys Gly Gln Gly Glu
   1 5
<210> 13
   <211> 15
   <212> PRT
   <213> Human rhinovirus
<400> 13
   Val Lys Ala Glu Thr Arg Leu Asn Pro Asp Leu Gln Pro Thr Glu
   1               5                   10                  15

## Description

### TECHNICAL FIELD

The present invention relates to methods for the assembly of nanostructures containing pilin proteins, to pilin protein assembly units for use in the construction of such nanostructures, and to nanostructures containing pilin protein assembly units.

### BACKGROUND OF THE INVENTION

Nanostructures are structures with individual components having one or more characteristic dimensions in the nanometer range (from about 1-100 nm). The advantages of assembling structures in which components have physical dimensions in the nanometer range have been discussed and speculated upon by scientists for over forty years. The advantages of these structures were first pointed out by Feynman (1959, There's Plenty of Room at the Bottom, An Invitation to Enter a New Field of Physics (lecture), December 29, 1959, American Physical Society, California Institute of Technology, reprinted *in Engineering and Science,* February 1960, California Institute of Technology, Pasadena, CA) and greatly expanded on by Drexler (1986, Engines of Creation, Garden City, N.Y.: Anchor Press/Doubleday). These scientists envisioned enormous utility in the creation of architectures with very small characteristic dimensions. The potential applications of nanotechnology are pervasive and the expected impact on society is huge (e.g., 2000, Nanotechnology Research Directions: IWGN Workshop Report; Vision for Nanotechnology R & D in the Next Decade; eds. M.C. Roco, R.S. Williams and P. Alivisatos, Kluwer Academic Publishers). It is predicted that there will be a vast number of potential applications for nanoscale devices and structures including electronic and photonic components; medical sensors; novel materials; biocompatible devices; nanoelectronics and nanocircuits; and computer technology.

The physical and chemical attributes of a nanostructure depend on the building blocks from which it is made. For example, the size of these building blocks, and the angles at which they join plays an important role in determining the properties of the nanostructure, and the positions in which functional elements can be placed. The art provides numerous examples of different types of materials which can be used in nanostructures, including DNA (US Patents Nos. 5,468,851, 5,948,897 and 6,072,044; WO 01/00876), bacteriophage T-even tail fibers (US Patents Nos. 5,864,013 and 5,877,279 and WO 00/77196), self-aligning peptides modeled on human elastin and other fibrous proteins (US Patent No.5,969,106), and artificial peptide recognition sequences (US Patent No. 5,712,366).

In Molecular Microbiology, 37 (5), 2000, pages 1146-1158 of Howell et al. as well as in WO 02/059156 hybrid proteins constructed from or containing two or more pilin proteins are disclosed.

Nevertheless, there is a continuing need for additional types of building blocks to provide the diversity which may be required to meet all of the potential applications for nanostructures. The present application provides a further class of building blocks which can be used in homogeneous nanostructures containing building blocks of only this class, or in heterogeneous nanostructures in combination with building blocks of other classes.

### SUMMARY OF THE INVENTION

The present invention provides nanostructures formed from a plurality of species of assembly units. With some exceptions, such as capping units, these assembly units comprise a plurality of different joining elements. In the nanostructures of the invention, the nanostructure includes at least one species of assembly unit in which at least one joining element comprises a pilin protein or binding derivative or binding fragment thereof. The pilin-containing assembly units may have two pilin-derived joining elements. In addition, the pilin containing assembly units may contain structural elements, functional elements or both.

The nanostructure of the invention is suitably prepared using a staged assembly method. In this method, a nanostructure intermediate comprising at least one unbound joining element is contacted with an assembly unit comprising a plurality of different joining elements, wherein:
(i) none of the joining elements of said plurality of different joining elements can interact with itself or with another joining element of said plurality, and
(ii) a single joining element of said plurality and a single unbound joining element of the nanostructure intermediate are complementary joining element.
   As a result, the assembly unit is non-covalently bound to the nanostructure intermediate to form a new nanostructure intermediate for use in subsequent cycles;
   (b) removing unbound assembly units; and
   (c) repeating steps (a) and (b) for a sufficient number of cycles to form a nanostructure.
   In the method of the invention, the complementary joining elements in at least one cycle comprise a pilin protein or a binding derivative or binding fragment thereof.

The invention further provides hybrid pilin proteins which are useful in forming the nanostructures of the invention. The hybrid pilin protein comprises the pilin amino terminal extension of a first pilin protein and the pilin protein body of a second pilin protein and lacks the pilin protein body of the first pilin protein and the pilin amino terminal extension of the second pilin protein, wherein the amino terminal extension of the first pilin protein does not bind to the pilin protein body of the second pilin protein.

### BRIEF DESCRIPTION OF THE DRAWINGS

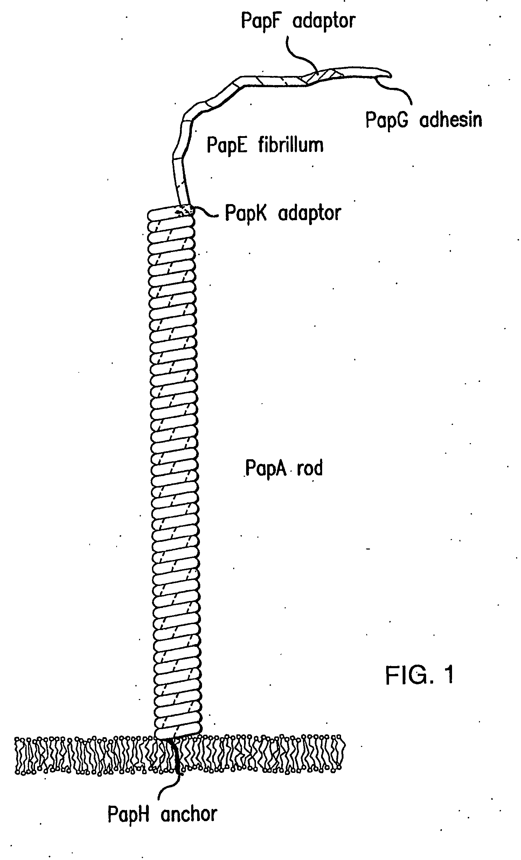

FIG. 1. Diagram of the structure of a P-pilus. The pilus is anchored to the outer membrane of *E. coli* through an N-terminal membrane anchor in papH. Most of the pilus is made up of many copies of papA. The rod is terminated by a single copy of papK that acts as an adaptor between the rod structure and a thin, distal structure called a fibrillum. The fibrillum consists of a few copies of papE, followed by a single copy of papF and a single copy of papG, which acts as the adhesin at the distal tip of the structure.

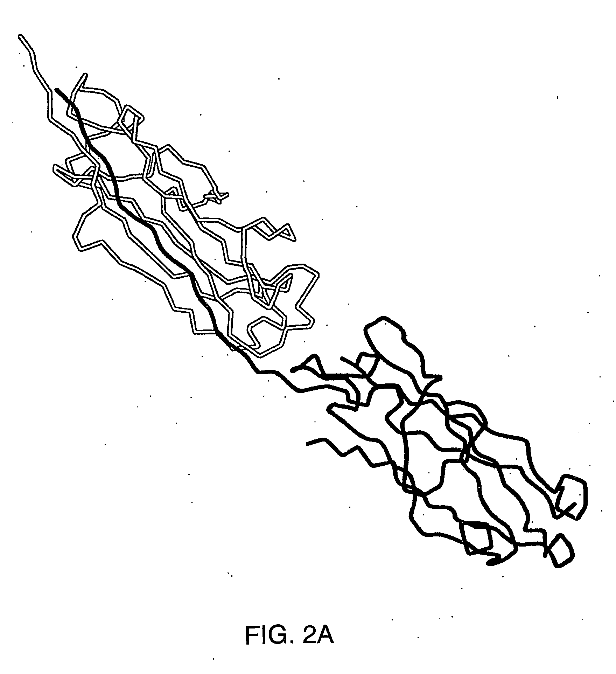

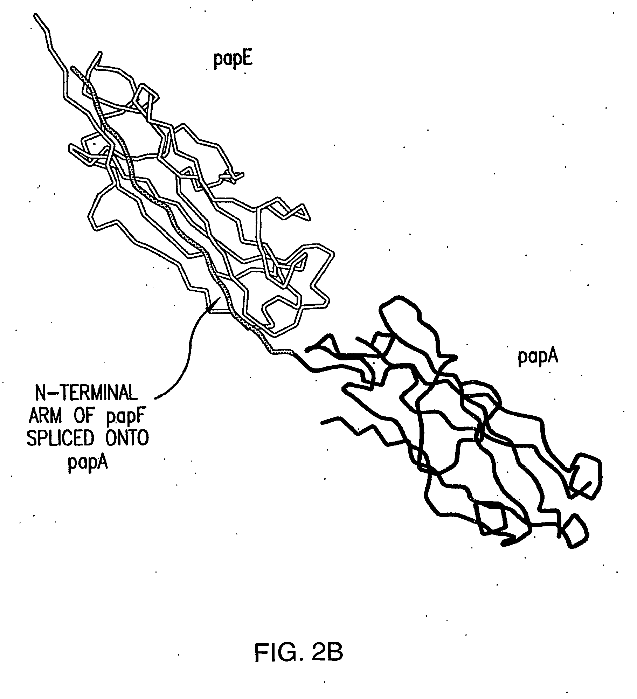

FIGS. 2 (A-B). A. Diagram of the interaction of two pilins, showing the close interaction of the N-terminal extension of one pilin (depicted in the lower right of the figure) with the groove on the surface of the other pilin (depicted in the upper left of the figure). Pilins interact through the binding of a long N-terminal extension from the pilin to the body of an adjacent pilin. This provides an extended, specific interaction with significant mechanical strength. B. Diagram of the interaction of papE with a hybrid pilin constructed from the N-terminal arm of papF spliced onto the protein body of papA. Replacing the N-terminal arm of a pilin with the N-terminal arm of a different pilin alters its binding specificity. Here, papA has had its N-terminal arm replaced by that of papF (arrow), now making it possible for the papA to interact with papE through the use of interactions normally used to stabilize the papF-papE interaction

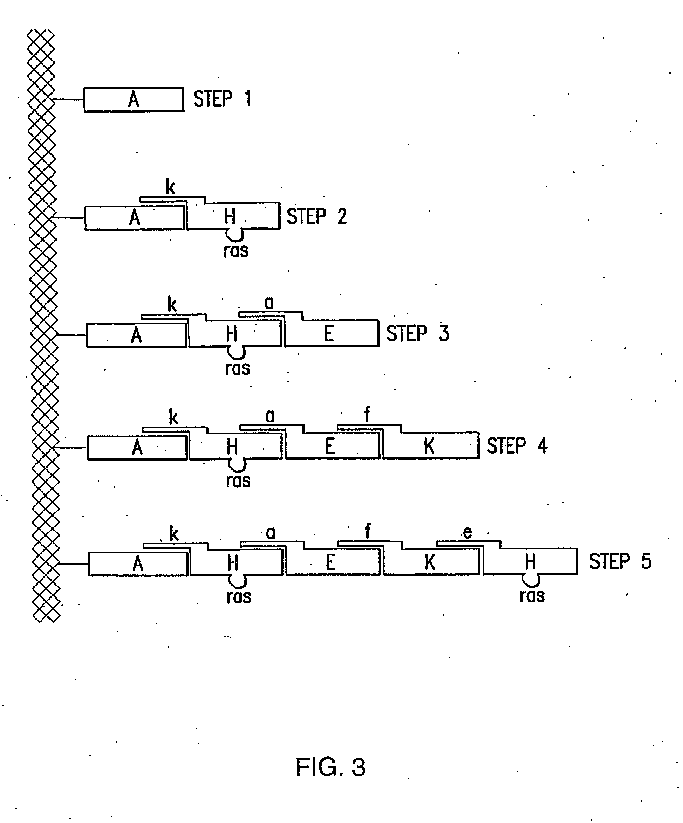

FIG. 3. Diagram of a staged assembly of hybrid pilin subunits. The illustrated process is described in Example 1. The addition of hybrid pilin subunits proceeds according to the steps indicated in the diagram. Hybrid pilins are made up of the protein body of one pilin (designated in capital letters, *e.g.*, A, H, E, K) and the N-terminal extension of another pilin (designated in lower-case letters, *e.g.,* k, a, f, e). The positioning of the ras epitope is indicated.

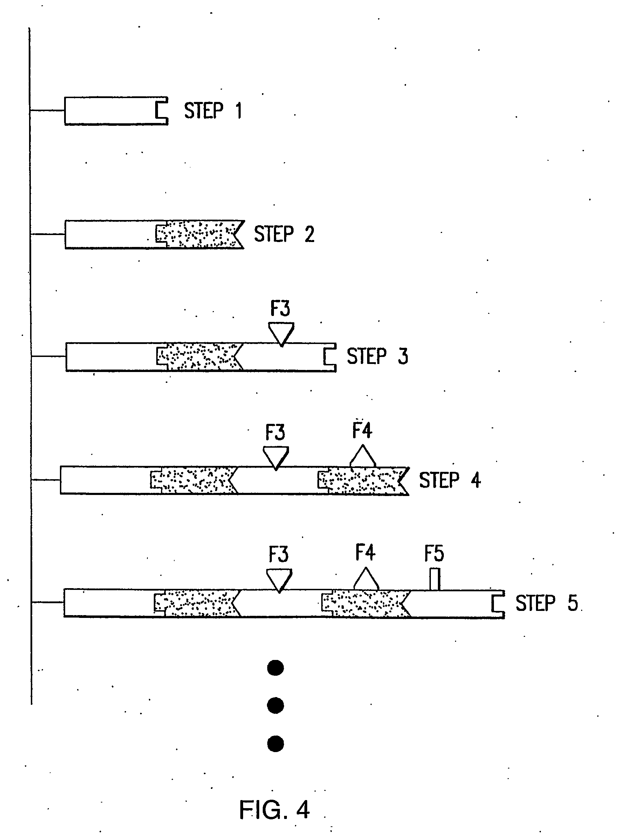

FIG. 4. Staged assembly of assembly units. In practice, each step in the staged assembly will be carried out in a massively parallel fashion. In step 1, an initiator unit is immobilized on a solid substrate. In the embodiment of the invention illustrated here, the initiator unit has a single joining element. In step 2, a second assembly unit is added. The second unit has two non-complementary joining elements, so that the units will not self-associate in solution. One of the joining elements on the second assembly unit is complementary to the joining element on the initiator unit. Unbound assembly units are washed away between each step (not shown).
   After incubation, the second assembly unit binds to the initiator unit, resulting in the formation of a nanostructure intermediate made up of two assembly units. In step 3, a third assembly unit is added. This unit has two non-complementary joining elements, one of which is complementary to the only unpaired joining element on the nanostructure intermediate. This unit also has a functional unit ("F3").
   A fourth assembly unit with functional element "F4" and a fifth assembly unit with functional element "F5"are added in steps 4 and 5, respectively, in a manner exactly analogous to steps 2 and 3. In each case, the choice of joining elements prevents more than one unit from being added at a time, and leads to a tightly controlled assembly of functional units in pre-designated positions.

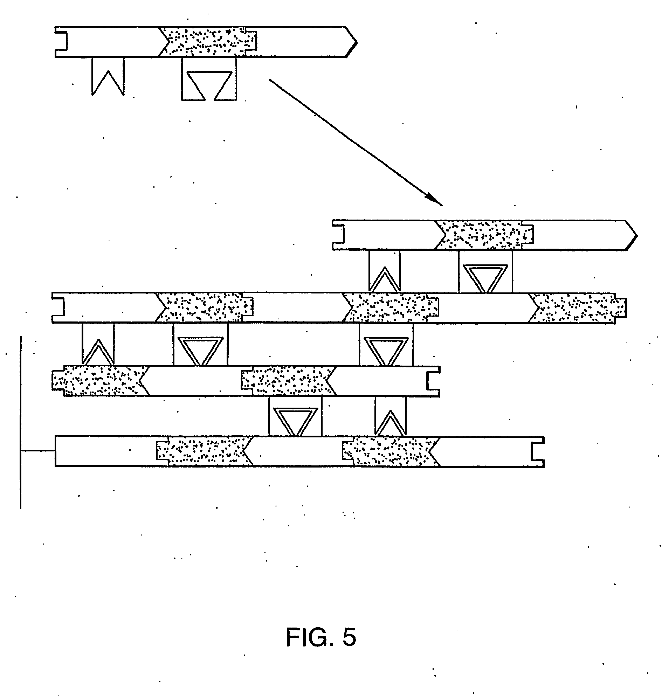

FIG. 5. Generation of a nanostructure from subassemblies. A nanostructure can be generated through the sequential addition of subassemblies, using steps analogous to those used for the addition of individual assembly units as illustrated above in FIG. 4. The arrow indicates the addition of a subassembly to a growing nanostructure.

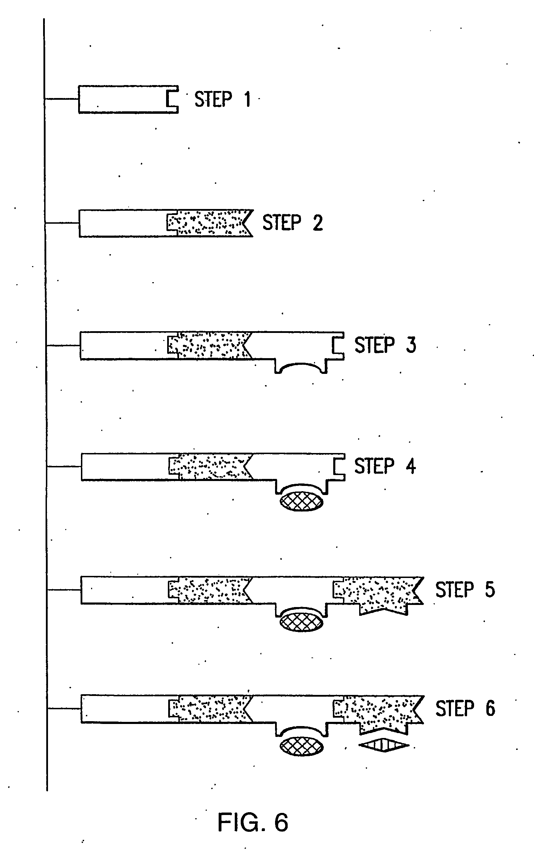

FIG. 6. A diagram illustrating the addition of protein units and inorganic elements to a nanostructure according to the staged assembly methods of the invention. In step 1, an initiator unit is bound to a solid substrate. In step 2, an assembly unit is bound specifically to the initiator unit. In step 3, an additional assembly unit is bound to the nanostructure undergoing assembly. This assembly unit comprises an engineered binding site specific for a particular inorganic element. In step 4, the inorganic element (depicted as a cross-hatched oval) is added to the structure and bound by the engineered binding site. Step 5 adds another assembly unit with a binding site engineered for specificity to a second type of inorganic element, and that second inorganic element (depicted as a hatched diamond) is added in step 6.

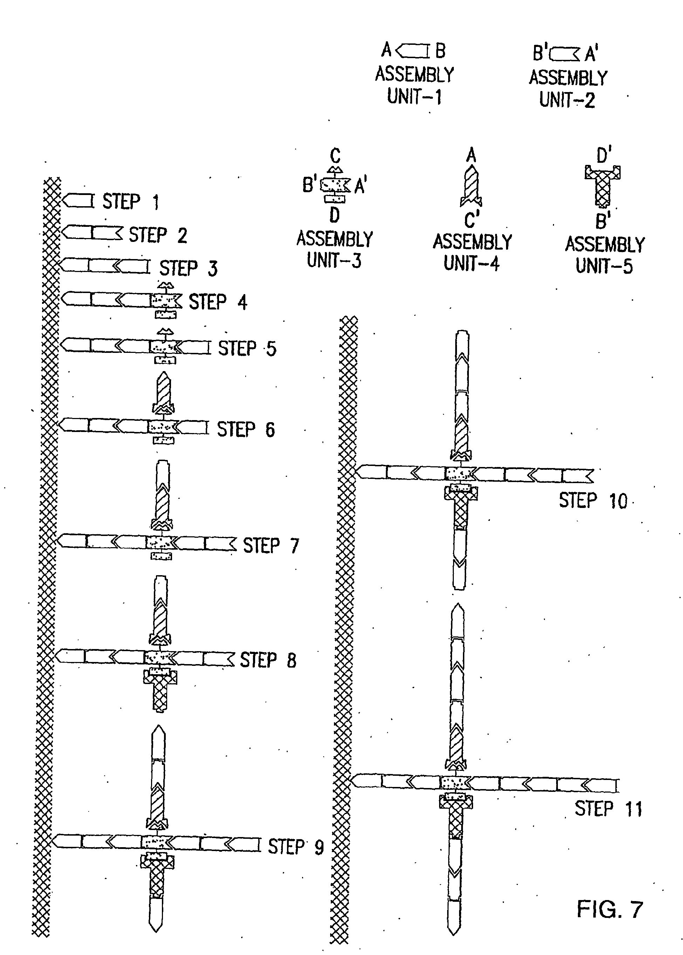

FIG. 7. Diagram of 11 steps of a staged assembly that utilizes four bispecific assembly units and one tetraspecific assembly unit to make a two-dimensional nanostructure.

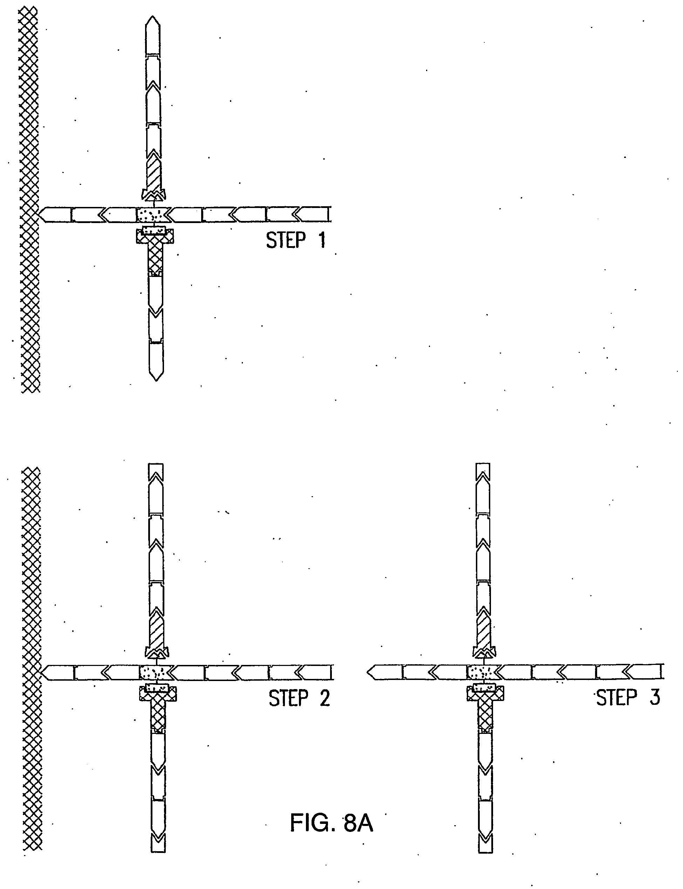

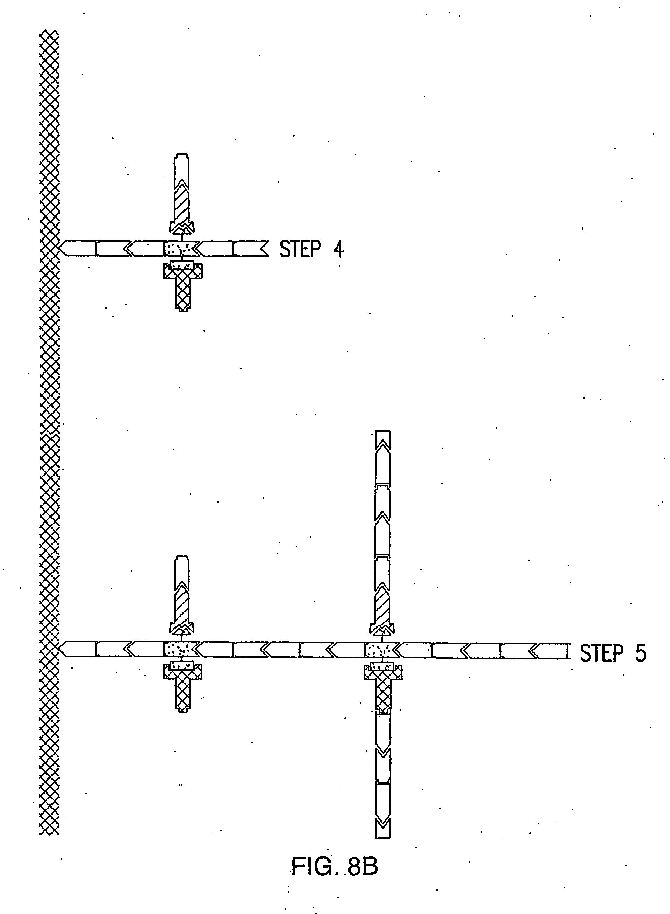

FIGS. 8(A-B). Diagram of a staged assembly that utilizes nanostructure intermediates as subassemblies. In Steps 1-3, a nanostructure intermediate is constructed, two joining elements are capped and the nanostructure intermediate is released from the solid substrate. In Step 5, the nanostructure intermediate from Step 3 is added to an assembly intermediate (shown in Step 4 attached to the solid substrate) as an intact subassembly.

### DETAILED DESCRIPTION OF THE INVENTION

**DEFINITIONS:** The terms in this application are generally used in a manner consistent with their ordinary meaning in the art. To provide clarity, however, in the event of a disagreement in the art, the following definitions control.

**Assembly Unit:** An assembly unit is an assemblage of atoms and/or molecules comprising structural elements, joining elements and/or functional elements. Preferably, an assembly unit is added to a nanostructure as a single unit through the formation of specific, non-covalent interactions. An assembly unit may comprise two or more sub-assembly units. An assembly unit may comprise one or more structural elements, and may further comprise one or more functional elements and one or more joining elements. If an assembly unit comprises a functional element, that functional element may be attached to or incorporated within a joining element or, in certain embodiments, a structural element. Such an assembly unit, which may comprise a structural element and one or a plurality of non-interacting joining elements, may be, in certain embodiments, structurally rigid and have well-defined recognition and binding properties.

**Assembly Unit, Initiator:** An initiator assembly unit is the first assembly unit incorporated into a nanostructure that is formed by the staged assembly method of the invention. It may be attached, by covalent or non-covalent interactions, to a solid substrate or other matrix as the first step in a staged assembly process. An initiator assembly unit is also known as an "initiator unit."

**Bottom-up:** Bottom-up assembly of a structure (*e.g.*,a nanostructure) is formation of the structure through the joining together of substructures using, for example, self-assembly or staged assembly.

**Capping Unit:** A capping unit is an assembly unit that comprises at most one joining element. Additional assembly units cannot be incorporated into the nanostructure through interactions with the capping unit once the capping unit has been incorporated into the nanostructure.

**Binding Derivative:** A derivative of a specified protein, for example a pilin protein, that retains the binding function of the specified protein but which is altered with respect to the base protein as a result of changes to the primary sequence (1) in which functionally equivalent amino acid residues are substituted for residues within the sequence resulting in a silent change, or (2) that produce a protein or peptide regions that is substantially homologous to the pilin protein of interest or a binding fragment thereof (e.g., in various embodiments, at least 60% or 70% or 80% or 90% or 95% identity over an amino acid sequence of identical size or when compared to an aligned sequence in which the alignment is done by a computer homology program known in the art) or whose encoding nucleic acid is capable of hybridizing to a sequence encoding the protein of interest, under highly stringent or moderately stringent conditions.

Functionally equivalent amino acid residues within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid.

**Binding Fragment:** A binding fragment of a molecule such as a peptide epitope or pilin protein is a fragment that exhibits the binding specificity of the peptide epitope or pilin protein from which the binding fragment is derived.

**First Assembly Unit, First Element**: For clarity, assembly units or elements are sometimes referred to using labels such as "first" or "second". This is purely a labeling convention and in no way indicates the position of the referenced assembly unit or element within the nanostructure.

**Functional Element:** A functional element is a moiety exhibiting any desirable physical, chemical or biological property that may be built into, bound or placed by specific covalent or non-covalent interactions, at well-defined sites in a nanostructure. Alternatively, a functional element can be used to provide an attachment site for a moiety with a desirable physical, chemical, or biological property. Examples of functional elements include, without limitation, a peptide, protein (e.g., enzyme), protein domain, small molecule, inorganic nanoparticle, atom, cluster of atoms, magnetic, photonic or electronic nanoparticles, or a marker such as a radioactive molecule, chromophore, fluorophore, chemiluminescent molecule, or enzymatic marker. Such functional elements can also be used for cross-linking linear, one-dimensional nanostructures to form two-dimensional and three-dimensional nanostructures.

**Joining Element:** A joining element is a portion of an assembly unit that confers binding properties on the unit, including, but not limited to: binding domain, hapten, antigen, peptide, PNA, DNA, RNA, aptamer, polymer or other moiety, or combination thereof, that can interact through specific, non-covalent interactions, with another joining element.

**Joining Elements, Complementary:** Complementary joining elements are two joining elements that interact with one another through specific, non-covalent interactions.

**Joining Elements, Non-Complementary:** Non-complementary joining elements are two joining elements that do not specifically interact with one another, nor demonstrate any tendency to specifically interact with one another.

**Joining Pair:** A joining pair is two complementary joining elements.

**Nanocomponent:** A nanocomponent is a substructure or portion of a nanostructure.

**Nanomaterial:** A nanomaterial is a material made up of a crystalline, partially crystalline or non-crystalline assemblage of nanoparticles.

**Nanoparticle:** A nanoparticle is an assemblage of atoms or molecules, bound together to form a structure with dimensions in the nanometer range (1-1000 nm). The , particle may be homogeneous or heterogeneous. Nanoparticles that contain a single crystal domain are also called nanocrystals.

**Nanostructure or Nanodevice:** A nanostructure or nanodevice is an assemblage of atoms and/or molecules comprising assembly units, i.e., structural, functional and/or joining elements, the elements having at least one characteristic length (dimension) in the nanometer range, in which the positions of the assembly units relative to each other are established in a defined geometry. The nanostructure or nanodevice may also have functional substitutents attached to it to provide specific functionality.

**Nanostructure intermediate:** A nanostructure intermediate is an intermediate substructure created during the assembly of a nanostructure to which additional assembly units can be added. In the final step, the intermediate and the nanostructure are the same.

**Non-covalent Interaction, Specific:** A specific non-covalent interaction is, for example, an interaction that occurs between an assembly unit and a nanostructure intermediate.

**Protein:** In this application, the term "protein" is used generically to referred to peptides, polypeptides and proteins comprising a plurality of amino acids, and is not intended to imply any minimum number of amino acids.

**Removing:** Removing of unbound assembly is accomplished when they are rendered unable to participate in further reactions with the growing nanostructure, whether or not they are physically removed.

**Self-assembly:** Self-assembly is spontaneous organization of components into an ordered structure. Also known as auto-assembly.

**Staged Assembly of a Nanostructure:** Staged assembly of a nanostructure is a process for the assembly of a nanostructure wherein a series of assembly units are added in a pre-designated order, starting with an initiator unit that is typically immobilized on a solid matrix or substrate. Each step results in the creation of an intermediate substructure, referred to as the nanostructure intermediate, to which additional assembly units can then be added. An assembly step comprises (i) a linking step, wherein an assembly unit is linked to an initiator unit or nanostructure intermediate through the incubation of the matrix or substrate with attached initiator unit or nanostructure intermediate in a solution comprising the next assembly units to be added; and (ii) a removal step, e.g., a washing step, in which excess assembly units are removed from the proximity of the intermediate structure or completed nanostructure. Staged assembly continues by repeating steps (i) and (ii) until all of the assembly units are incorporated into the nanostructure according to the desired design of the nanostructure. Assembly units bind to the initiator unit or nanostructure intermediate through the formation of specific, non-covalent bonds. The joining elements of the assembly units are chosen so that they attach only at pre-designated sites on the nanostructure intermediate. The geometry of the assembly units, the structural elements, and the relative placement of joining elements and functional elements, and the sequence by which assembly units are added to the nanostructure are all designed so that functional units are placed at pre-designated positions relative to one another in the structure, thereby conferring a desired function on the completely assembled nanostructure.

**Structural Element:** A structural element is a portion of an assembly unit that provides a structural or geometric linkage between joining elements, thereby providing a geometric linkage between adjoining assembly units. Structural elements provide the structural framework for the nanostructure of which they are a part.

**Subassembly:** A subassembly is an assemblage of atoms or molecules consisting of multiple assembly units bound together and capable of being added as a whole to an assembly intermediate (e.g., a nanostructure intermediate). In many embodiments of the invention, structural elements also support the functional elements in the assembly unit.

**Top-down**: Top-down assembly of a structure (e.g.,a nanostructure) is formation of a structure through the processing of a larger initial structure using, for example, lithographic techniques.

### Pilin Assembly Units

The present invention provides a new class of assembly units that can be used in production of nanostructures. These "pilin assembly units" contain at least one joining element that is a pilin protein or a binding derivative or fragment thereof. In addition, the assembly unit may contain structural elements (which may themselves be part of a pilin protein) and/or functional elements.

Pilins are the protein units making up bacterial adhesion pili. Bacterial adhesion pili ("P-pili") are formed by the polymerization of pilins (*see, e.g.,* Bullitt and Makowski, 1995, Structural polymorphism of bacterial adhesion pili, Nature 373: 164-67; Bullitt and Makowski, 1998, Bacterial adhesion pili are heterologous assemblies of similar units, Biophysics J. 74: 623-32). Pili units may be assembled *in vitro* (*see, e.g.,* Bullitt *et al*., 1996, Development of pilus organelle sub-assemblies in vitro depends on chaperone uncapping of a beta zipper, Proc. Nat. Acad. Sci. USA 93: 12890-95).

P-pili expressed on the surface of *E. coli* are helical filaments 6.8 nm in diameter, with an ellipsoidal central cavity 2.5 nm x 1.5 nm that winds about the helical axis, connecting to radial channels that extend to the surface of the pili (Hultgren and Normark, 1991, Biogenesis of the bacterial pilus, Curr. Opin. Genet. Dev. 1: 313-18; Hultgren *et al.,* 1993, Pilus and nonpilus bacterial adhesins: assembly and function in cell recognition, Cell 73(5): 887-901; Bullitt and Makowski, 1995, Structural polymorphism of bacterial adhesion pili, Nature 373: 164-67). Each pilus comprises approximately 1000 copies of the major pilin, PapA, and one or a few copies of the minor pilins, PapH, PapK, PapE, PapF, and PapG. In the PapA-containing coiled rod region of the helix, there are 3.29 subunits per turn of the helix, with a 7.6 Å rise per subunit (Bullitt and Makowski, 1995, Structural polymorphism of bacterial adhesion pili, Nature 373: 164-167). The fibrillae at the distal tip of the pilus is made up of four distinct but homologous pilins (Fig. 1). The distal end of papA will interact with the proximal end of papA or papK. The proximal end of papK will interact only with papA; its distal end only with papE; and so on as required by its remarkable architecture. These specific interactions are summarized in Table 1.

| **Table 1: Pilin-Pilin Protein Interactions** | | |
|---|---|---|
| Pilin | End | Interacts with Pilin |
| papA | Proximal | papA and papH |
| papA | Distal | pap A and papK |
| papH | Proximal | none |
| pap H | Distal | papA |
| papK | Proximal | papA |
| papK | Distal | papE |
| papE | Proximal | papH and papE |
| papE | Distal | papE and papF |
| papF | Proximal | papE |
| papF | Distal | papG |
| papG | Proximal | papF |
| papG | Distal | Does not interact with the |
| | | N-terminal extension of any |
| | | papA, papH, papE, papF, or |
| | | papG |

These natural binding affinities are exploited in the nanostructures and method of the present invention. The interaction between pilin proteins is mediated by the N-terminal extension of each pilin protein that binds to the immediately adjacent pilin protein in P-pili, yielding an extended intermolecular interface that provides significant mechanical strength to the pilus (Sauer *et al*., 1999, Structural basis of chaperone function and pilus biogenesis, Science 285: 1058-61; Choudhury *et al.,* 1999, X-ray structure of the FimC-FimH chaperone-adhesin complex from uropathogenic Escherichia coli, Science 285: 1061-66). The affinity and specificity of this binding are determined by the interaction between the extended N-terminal arm and the groove on the adjacent pilin protein (Fig. 2A). Consequently, replacement of the N-terminal arm of one pilin with that from another (forming a "hybrid" or "chimeric" pilin protein) provides a means of altering the specificity of binding of a pilin unit, and allows the design of pilin assembly units.

Pilins are highly homologous in the region spanning the C-terminal end of their N-terminal extension and the N-terminal end of the pilin body. This region of homology provides guidance for the design of hybrid pilins made of the N-terminal extension of one pilin and the body of the other. A hybrid pilin comprises the N-terminal extension from one pilin and the body of another pilin, and may be used as structural and/or joining elements in staged assembly where the N-terminus and body of one pilin do not interact to form dimers and polymers (FIG. 2B). A comparison of the sequences of all the pilins that make up a P-pilus indicate that the region that links the N-terminal extension with the body of that pilin protein is highly conserved among pilins and that the position for fusing heterologous pilin parts is well-defined based on that homology.

Non-limiting examples of the N-terminal extensions of various pilin proteins, and the N-terminal amino acid sequences of various pilin protein bodies lacking the N-terminal extension, are shown in Table 2, below. Hybrid pilins that comprise the N-terminal extension from one pilin and the body of another pilin, may be expressed and purified by methods commonly known in the art (e.g., Bullitt and Makowski, 1995, Structural polymorphism of bacterial adhesion pili, Nature 373: 164-67; Bullitt *et al.,* 1996, Development of pilus organelle sub-assemblies in vitro depends on chaperone uncapping of a beta zipper, Proc. Natl. Acad. Sci. USA 93: 12890-95).

| **Table 2: Amino Acid Sequence of the N-terminal Extension and the Adjacent Pilin Body of Pilins papA, papK, papH, papE, and papF** | | |
|---|---|---|
| Pilin | N-Terminal Extension | N-Terminal Amino Acid Sequence of the Pilin Protein Body Lacking the N-terminal Extension |
| papA: | AVPQGQGKVTFSGTVVDA | PCGIDAAQSADQSVDFGQISK |
| | (SEQ ID NO: 1) | VFLDNDGQTTPKAFDIKLVNC |
| | | DITNYKKPATG |
| | | (SEQ ID NO: 2) |
| papK: | MIKSTGALLLFAALSAGQAIASDVAFR | PCHVSGDSLNKHVVFKTRAS |
| | GNLLDR | RDFWYPPGRSPTESFVI |
| | (SEQ ID NO: 3) | (SEQ ID NO: 4) |
| papH: | MRLRFSVPLFFFGCVFVHGVFAGPFPPP | PACTLAMEDAWQIID |
| | GMSLPEYWGEEHVWWDGRAAFHGEV | (SEQ ID NO: 6) |
| | VR | |
| | (SEQ ID NO: 5) | |
| papE: | MKKIRGLCLPVMLGAVLMSQHVHAAD | PACTVTKAEVDWGNVEIQTLS |
| | NLTFKGKLII | PDGSRHQKDFSVG |
| | (SEQ ID NO: 7) | (SEQ ID NO: 8) |
| papF: | MARLSLFISLLLTSVAVLADVQINIRGN | PPCTINNGQNIVVDFGNINPEH |
| | VYI | VDNSRGEITKTISISCT |
| | (SEQ ID NO: 9) | (SEQ ID NO: 10) |

As shown in Table 2 above, the N-terminal extension of papA comprises the first 20 amino acids. The extension is longer in other pilins. PapH has a particularly long extension because it is required for anchoring to the outer membrane of *E. coli.* Consequently, this long papH extension is not used in preferred embodiments of the present invention. It is included in Table 2 to illustrate the preservation among pilins of the sequences in the region that comprises the second half of the N-terminal extension, and in the region of the N-terminal portion of the pilin protein body. Similarities in the N-terminal extensions indicate that the extensions are used in the same way and interact with the proximal pilin in similar fashion. Differences in the sequences of the N-terminal extensions are responsible for the differences in their binding specificity.

Pilin assembly units can be fabricated that comprises fragment of multiple pilin proteins, wherein each pilin unit comprises a joining element that is a peptide epitope. Recognition of such epitopes by a cognate antibody provides one method for utilizing pilin proteins as joining elements. Preferably, however, the joining elements of one assembly unit is a pilin N-terminal peptide domain that is recognized and bound by certain other pilin proteins, as described above. The use of joining pair comprising a pilin N terminal domain and a pilin C-terminal domain provides options in the construction of nanostructures because the joining elements of such assembly units are not fully rigid prior to assembly and therefore permit protein-protein interactions that are flexible prior to binding. A pilin protein recognizes and binds to the flexible extension of another pilin protein, and thus can serve as a joining element suitable for use in the staged assembly of nanostructures according to the present invention. After binding, the N-terminal extension is held rigidly through its binding to an adjacent, cognate pilin protein, providing the rigidity needed in a staged assembly. Thus, pilin protein can function as both joining element and structural element in the same assembly unit.

Generally, a pilin protein fragment is unlikely to maintain its structure adequately to provide for specific and tight interactions with other pilin proteins, unless that fragment comprises substantially all of the pilin protein. However, in certain embodiments of the invention, a few amino acids may be altered, added, or deleted to one or more of the beta turns of the pilin without disrupting its overall structure, structural rigidity or recognition properties, thereby providing one or more sites suitable for the insertion of a functional element. Functionality may be added to the pilin subunits at positions identified as being (i) on the surface of the subunits; (ii) unimportant to the interaction of the subunits with one another and (iii) unimportant for the stability of the subunits themselves. It has been shown that in many proteins, large loop insertions are tolerated and many redesigns have generated proteins that successfully fold to stable, active structures. Some redesigns have been entirely the choice of the investigators, whereas others have incorporated a randomization and selection step to identify optimal sequences (Regan, 1999, Protein redesign, Current Opinion in Structural Biology 9: 494-99). One region amenable to reengineering is a surface loop on papA comprised of gly107-ala108-gly109. This loop satisfies the criteria that must be met by a position where a heterologous peptide may be successfully inserted. Determining the position of surface loops in a protein is carried out by examination of the three-dimensional structure of the protein or a homolog thereof, if three-dimensional atomic coordinates are available, using, for example, a public-domain protein visualization computer program such as RASMOL (Sayle *et al*., 1995, RasMol: Biomolecular graphics for all, Trends Biochem. Sci. (TIBS) 20(9): 374-376; Saqi *et al*., 1994, PdbMotif -a tool for the automatic identification and display of motifs in protein structures, Comput. Appl. Biosci. 10(5): 545-46). In this manner, amino acids included in surface loops, and the relative spatial locations of these surface loops, can be determined.

The following are non-limiting examples of hybrid pilin assembly units that may be engineered for use in the compositions and methods of the invention:
(i) PapH with the amino terminus of papK. Using standard methods, the DNA sequence coding for the amino terminal extension of papH is replaced with the DNA sequence encoding the amino terminal extension of papK within a plasmid designed to overproduce papH.
(ii) PapH-papK hybrid with added epitope: DNA coding for a Ras epitope (EEYSAMRDQYMRTGEL; SEQ ID No.: 11, recognized by monoclonal antibody Y13-259) is inserted in the gene for papH between the two codons coding for amino acids 121 and 126 of papH (at the position corresponding to the surface loop in papA). Determining the position of surface loops in a protein is carried out by examination of the three-dimensional structure of the protein or a homolog thereof, if three-dimensional atomic coordinates are available, using, for example, a public-domain protein visualization computer program such as RASMOL (Sayle *et al.,* 1995, RasMol: Biomolecular graphics for all, Trends Biochem. Sci. (TIBS) 20(9): 374-376; Saqi *et al.,* 1994, PdbMotif--a tool for the automatic identification and display of motifs in protein structures, Comput. Appl. Biosci. 10(5): 545-46). In this manner, amino acids included in surface loops, and the relative spatial locations of these surface loops, can be determined.
(iii) PapE with the amino terminus of papA: Using standard methods of recombinant DNA technology, the DNA sequence coding for the amino terminal extension of papE is replaced with the DNA sequence encoding the amino terminal extension of papA within a plasmid designed to overproduce papE.
(iv) PapK with the amino terminus of papF: Using standard methods of recombinant DNA technology, the DNA sequence coding for the amino terminal extension of papK is replaced with the DNA sequence encoding the amino terminal extension of papF within a plasmid designed to overproduce papK.
(v) PapH with the amino terminus of papE. Using standard methods of recombinant DNA technology, the DNA sequence coding for the amino terminal extension of papH is replaced with the DNA sequence encoding the amino terminal extension of papE within a plasmid designed to overproduce papH.
(vi) PapH-papE hybrid with added epitope: DNA coding for a Ras epitope is inserted in the gene for papH between the two codons coding for amino acids 121 and 126 of papH (at the position corresponding to the surface loop in papA).

Pilin proteins may be expressed and purified by methods commonly known in the art (*e*.*g*., Bullitt and Makowski, 1995, Structural polymorphism of bacterial adhesion pili, Nature 373: 164-67; Bullitt *et al*., 1996, Development of pilus organelle sub-assemblies in vitro depends on chaperone uncapping of a beta zipper, Proc. Natl. Acad. Sci. USA 93: 12890-95). If the three-dimensional structure of the protein being engineered is not known, but that of a close homolog is known (as is the case, for example, for essentially all antibody molecules), the amino acid sequence of the molecule of interest, or a portion thereof, can be aligned with that of the molecule whose three-dimensional structure is known. This comparison (done, for example, using BLAST (Altschul *et al*., 1997, Gapped BLAST and PSI-BLAST: a new generation of protein database search programs, Nucleic Acids Res. 25: 3389-3402) or LALIGN (Huang and Miller, 1991, A time efficient, linear-space local similarity algorithm, Adv. Appl. Math. 12: 337-357) allows identification of all the amino acids in the protein of interest that correspond to amino acids that constitute surface loops (β-turns) in the protein of known three-dimensional structure. In regions in which there is high sequence similarity between the two proteins, this identification is carried out with a high level of certainty. Once a putative loop is identified and altered according to methods disclosed herein, the resultant construct is tested to determine if it has the expected properties. This analysis is performed even in those instances where identification of the loop is highly reliable, *e.g.* where that determination is based upon a known three-dimensional protein structure.

In certain embodiments of the present invention, an assembly unit comprises a structural element. As noted above, that structural element in a pilin assembly unit may be derived from a pilin protein. More generally, the structural element generally has a rigid structure (although in certain embodiments, described below, the structural element may be non-rigid). The structural element is preferably a defined peptide, protein or protein fragment of known size and structure that comprises at least about 50 amino acids and, generally, fewer than 2000 amino acids. Peptides, proteins and protein fragments are preferred since naturally-occurring peptides, proteins and protein fragments have well-defined structures, with structured cores that provide stable spatial relationships between and among the different faces of the protein. This property allows the structural element to maintain pre-designed geometric relationships between the joining elements and functional elements of the assembly unit, and the relative positions and stoichiometries of assembly units to which it is bound.

The use of proteins as structural elements has particular advantages over other choices such as inorganic nanoparticles. Most populations of inorganic nanoparticles are heterogeneous, making them unattractive scaffolds for the assembly of a nanostructure. In most populations, each inorganic nanoparticle is made up of a different number of atoms, with different geometric relationships between facets and crystal faces, as well as defects and impurities. A comparably sized population of proteins is, by contrast, very homogeneous, with each protein comprised of the same number of amino acids, each arranged in approximately the same way, differing in arrangement, for the most part, only through the effect of thermal fluctuations. Consequently, two proteins designed to interact with one another will always interact with the same geometry, resulting in the formation of a complex of predictable geometry and stoichiometry. This property is essential for massively parallel "bottom-up" assembly of nanostructures.

A structural element may be used to maintain the geometric relationships among the joining elements and functional elements of a nanostructure. As such, a rigid structural element is generally preferred for construction of nanostructures using the staged assembly methods described herein. This rigidity is typical of many proteins and may be conferred upon the protein through the properties of the secondary structural elements making up the protein, such as α-helices and β-sheets.

Structural elements may be based on the structure of proteins, protein fragments or peptides whose three-dimensional structure is known or may be designed *ab initio.* Examples of proteins or protein fragments that may be utilized as structural elements in an assembly unit include, but are not limited to, antibody domains, diabodies, single-chain antibody variable domains, and bacterial pilins.

In some embodiments, structural elements, joining elements and functional elements may be of well-defined extent, separated, for example, by glycine linkers. In other embodiments, joining elements may involve peptides or protein segments that are integral parts of a structural element, or may comprise multiple loops at one end of a structural element, such as in the case of the complementarity determining regions (CDRs) of antibody variable domains (Kabat *et al*., 1983, Sequences of Proteins of Immunological Interest, U.S. Department of Health and Human Services). A CDR is a joining element that is an integral part of the variable domain of an antibody. The variable domain represents a structural element and the boundary between the structural element and the CDR making up the joining element (although well-defined in the literature on the basis of the comparisons of many antibody sequences) may not always be completely unambiguous structurally. There may not always be a well-defined boundary between a structural element and a joining element, and the boundary between these domains, although well-defined on the basis of their respective utilities, may be ambiguous spatially.

Structural elements of the present invention comprise, e.g., core structural elements of naturally-occurring proteins that are then modified to incorporate joining elements, functional elements, and/or a flexible domain (e.g., a tri-, tetra- or pentaglycine), thereby providing useful assembly units. Consequently, in certain embodiments, structures of existing proteins are analyzed to identify those portions of the protein or part thereof that can be modified without substantially affecting the rigid structure of that protein or protein part.

The pilin assembly unit of the present invention include at least one pilin or a binding derivative or fragment thereof as a joining element. Additional joining elements in the pilin assembly unit may be any joining element that confers binding properties on the assembly unit including, but not limited to: pilin joining elements, haptens, antigens, peptides, PNAs, DNAs, RNAs, aptamers, polymers or other moieties, or combination thereof, that can interact through specific non-covalent interactions, with another joining element.

In certain embodiments, an assembly unit having more than two joining elements is used to build a nanostructure. The additional joining elements can be used, for example: (i) as an attachment point for addition or insertion of a functional element or functional moiety (see Table 1 above); (ii) as the attachment point of the initiator to a solid substrate; or (iii) as attachment points for subassemblies.

The pilin assembly unit used in the invention may also incorporate functional elements. Functional elements may be incorporated into assembly units and, ultimately into one-, two-, and three-dimensional nanostructures in such a manner as to provide well-defined spatial relationships between and among the functional elements. These well-defined spatial relationships between and among the functional elements permit them to act in concert to provide activities and properties that are not attainable individually or as unstructured mixtures.

In one aspect of the invention, functional elements include, but are not limited to, peptides, proteins, protein domains, small molecules, inorganic nanoparticles, atoms, clusters of atoms, magnetic, photonic or electronic nanoparticles. The specific activity or property associated with a particular functional element, which will generally be independent of the structural attributes of the assembly unit to which it is attached, can be selected from a very large set of possible functions, including but not limited to, a biological property such as those conferred by proteins (*e.g.*, a transcriptional, translational, binding, modifying or catalyzing property). In other embodiments, functional groups may be used that confer chemical, organic, physical electrical, optical, structural, mechanical, computational, magnetic or sensor properties to the assembly unit.

In another aspect of the invention, functional elements include, but are not limited to: metallic or metal oxide nanoparticles (Argonide Corporation, Sanford, FL; NanoEnergy Corporation, Longmont, CO; Nanophase Technologies Corporation, Romeoville, IL; Nanotechnologies, Austin, TX; TAL Materials, Inc., Ann Arbor, MI); gold or gold-coated nanoparticles (Nanoprobes, Inc., Yaphank, NY; Nanospectra LLC, Houston TX); immunoconjugates (Nanoprobes, Inc., Yaphank, NY); non-metallic nanoparticles (Nanotechnologies, Austin, TX); ceramic nanofibers (Argonide Corporation, Sanford, FL); fullerenes or nanotubes (*e.g.*, carbon nanotubes) (Materials and Electrochemical Research Corporation, Tucson, AZ; Nanolab, Brighton MA; Nanosys, Inc., Cambridge MA; Carbon Nanotechnologies Incorporated, Houston, TX); nanocrystals (NanoGram Corporation, Fremont, CA; Quantum Dot Corporation, Hayward CA); silicon or silicate nanocrystals or powders (MTI Corporation, Richmond, CA); nanowires (Nanosys, Inc., Cambridge MA); or quantum dots (Quantum Dot Corporation, Hayward CA; Nanosys, Inc., Cambridge MA).

Functional elements may also comprise any art-known detectable marker, including radioactive labels such as ³²P, ³⁵S, ³H, and the like; chromophores; fluorophores; chemiluminescent molecules; or enzymatic markers.

In certain embodiment of this invention, a functional element is a fluorophore. Exemplary fluorophore moieties that can be selected as labels are set forth in Table 3.

| **Table 3: Fluorophore Moieties That Can Be Used as Functional Elements** | |
|---|---|
| 4-acetamido-4'-isothiocyanatostilbene-2,2'disulfonic acid | |
| acridine and derivatives: | |
| | acridine |
| | acridine isothiocyanate |
| 5-(2'-aminoethyl)aminonaphthalene-1-sulfonic acid (EDANS) | |
| 4-amino-N-[3-vinylsulfonyl)phenyl]naphthalimide-3,5 disulfonate (Lucifer Yellow VS) | |
| -(4-anilino-1-naphthyl)maleimide | |
| anthranilamide | |
| Brilliant Yellow | |
| coumarin and derivatives: | |
| | coumarin |
| | 7-amino-4-methylcoumarin (AMC, Coumarin 120) |
| | 7-amino-4-trifluoromethylcoumarin (Coumarin 151) |
| Cy3 | |
| Cy5 | |
| cyanosine | |
| 4',6-diaminidino-2-phenylindole (DAPI) | |
| 5',5"-dibromopyrogallol-sulfonephthalein (Bromopyrogallol Red) | |
| 7-diethylamino-3-(4'-isothiocyanatophenyl)-4-methylcoumarin | |
| diethylenetriamine pentaacetate | |
| 4,4'-diisothiocyanatodihydro-stilbene-2,2'-disulfonic acid | |
| 4,4'-diisothiocyanatostilbene-2,2'-disulfonic acid | |
| 5-[dimethylamino]naphthalene-1-sulfonyl chloride (DNS, dansyl chloride) | |
| 4-(4'-dimethylaminophenylazo)benzoic acid (DABCYL) | |
| 4-dimethylaminophenylazophenyl-4'-isothiocyanate (DABITC) | |
| eosin and derivatives: | |
| | eosin |
| | eosin isothiocyanate |
| erythrosin and derivatives: | |
| | erythrosin B |
| | erythrosin isothiocyanate |
| ethidium | |
| fluorescein and derivatives: | |
| | 5-carboxyfluorescein (FAM) |
| | 5-(4,6-dichlorotriazin-2-yl)aminofluorescein (DTAF) |
| | 2'7'-dimethoxy-4'5'-dichloro-6-carboxyfluorescein (JOE) |
| | fluorescein |
| | fluorescein isothiocyanate |
| | QFITC (XRITC) |
| fluorescamine | |
| IR144 | |
| IR1446 | |
| Malachite Green isothiocyanate | |
| 4-methylumbelliferone | |
| ortho cresolphthalein | |
| nitrotyrosine | |
| pararosaniline | |
| Phenol Red | |
| B-phycoerythrin | |
| o-phthaldialdehyde | |
| pyrene and derivatives: | |
| | pyrene |
| | pyrene butyrate |
| | succinimidyl 1-pyrene butyrate |
| Reactive Red 4 (Cibacron® Brilliant Red 3B-A) | |
| rhodamine and derivatives: | |
| | 6-carboxy-X-rhodamine (ROX) |
| | 6-carboxyrhodamine (R6G) |
| | lissamine rhodamine B sulfonyl chloride |
| | rhodamine (Rhod) |
| | rhodamine B |
| | rhodamine 110 |
| | rhodamine 123 |
| | rhodamine X isothiocyanate |
| | sulforhodamine B |
| | sulforhodamine 101 |
| | sulfonyl chloride derivative of sulforhodamine 101 (Texas Red) |
| | N,N,N',N'-tetramethyl-6-carboxyrhodamine (TAMRA) |
| | tetramethyl rhodamine |
| | tetramethyl rhodamine isothiocyanate (TRITC) |
| riboflavin | |
| rosolic acid | |
| terbium chelate derivatives | |

In other embodiments, a functional element is a chemiluminescent substrate such as luminol (Amersham Biosciences), BOLD^{™} APB (Intergen), Lumigen APS (Lumigen), etc.

In another embodiment, the functional element is an enzyme. The enzyme, in certain embodiments, may produce a detectable signal when a particular chemical reaction is conducted, such as the enzymes alkaline phosphatase, horseradish peroxidase, β-galactosidase, etc.

In another embodiment, a functional element is a hapten or an antigen (e.g., ras). In yet another embodiment, a functional element is a molecule such as biotin, to which a labeled avidin molecule or streptavidin may be bound, or digoxygenin, to which a labeled anti-digoxygenin antibody may be bound.

In another embodiment, a functional element is a lectin such as peanut lectin or soybean agglutinin. In yet another embodiment, a functional element is a toxin, such as *Pseudomonas* exotoxin (Chaudhary *et al*., 1989, A recombinant immunotoxin consisting of two antibody variable domains fused to Pseudomonas exotoxin, Nature 339(6223): 394-97).

Peptides, proteins or protein domains may be added to proteinaceous assembly units using the tools of molecular biology commonly known in the art to produce fusion proteins in which the functional elements are introduced at the N-terminus of the proteins, the C-terminus of the protein, or in a loop within the protein in such a way as to not disrupt folding of the protein. Non-peptide functional elements may be added to an assembly unit by the incorporation of a peptide or protein moiety that exhibits specificity for said functional element, into the proteinaceous portion of the assembly unit.

In certain embodiments, a functional unit is attached by splicing a protein domain or peptide into the proteinaceous portion of an assembly unit. In such embodiments, the position for insertion must be chosen such that it does not disrupt the folding of the protein unit, since the binding specificity and affinity of the assembly unit will depend on the ability of the assembly unit to fold correctly. Also preferably, the site at which an insert is added does not cause disruption of the folding of the protein unit. Preferably, the site of insertion is a surface loop having little interaction with the remainder of the protein. When the three-dimensional structure of the protein is known, *e.g.*, in the case of the pilin papK, such sites may be identified by visual examination of the protein structure using a computer graphics program, such as RasMol (Sayle *et al.,* 1995, RasMol: Biomolecular graphics for all, Trends Biochem. Sci. (TIBS) 20(9): 374-76). The coordinates defining the three-dimensional positions of the atoms of papK are included in the PDB file 1PDK, which also provides the three-dimensional structure of the chaperone papD that is complexed with papK in the solved crystal structure. Upon such an analysis, it is apparent that there is a surface loop that includes residues 109-113 (sequence NKGQGE (SEQ ID NO: 12) according to the PDB file), which represents a site with high potential for accepting the insertion of a peptide such as the ras antigen.

In a specific embodiment, one or more functional elements is added to an assembly unit comprising a pilin protein at a position identified as being (i) on the surface of the unit; (ii) unimportant to the interaction of the unit with other pilin-comprising assembly unit; and (iii) unimportant for the stability of the unit itself. It has been shown that large loop insertions are tolerated and many recombinant proteins have been expressed that are able to fold successfully into stable, active protein structures. In some instances, such recombinant proteins have been designed and produced without further genetic manipulation, while other approaches have incorporated a randomization and selection step to identify optimal sequence alterations (Regan, 1999, Protein redesign, Curr. Opin. Struct. Biol. 9: 494-99). For example, one pilin region amenable to re-engineering is a surface loop on papA comprising the sequence gly107-ala108-gly109. This loop satisfies all the above-described criteria as a position at which a heterologous peptide may be inserted.

In another embodiment, an entire antibody variable domain (*e.g.* a single-chain variable domain) is incorporated into an assembly unit, *e.g.* into the joining or structural element thereof, in order to act as an affinity target for a functional element. In this embodiment, wherein an entire antibody variable domain is inserted into a surface loop of, *e.g.,* a joining element or a structural element, a flexible segment (*e.g.*, a polyglycine peptide sequence) is preferably added to each side of the variable domain sequence. This polyglycine linker will act as a flexible spacer that facilitates folding of the original protein after synthesis of the recombinant fusion protein. The antibody domain is chosen for its binding specificity for a functional element, which can be, but is not limited to, a protein or peptide, or to an inorganic material.

In another embodiment of the present invention, a functional element may be a quantum dot (semiconductor nanocrystal, *e.g.*, QDOT^{™}, Quantum Dot Corporation, Hayward, CA) with desirable optical properties. A quantum dot can be incorporated into a nanostructure through a peptide that has specificity for a particular class of quantum dot. As would be apparent to one of ordinary skill, identification of such a peptide, having a required affinity for a particular type of quantum dot, is carried out using methods well known in the art. For example, such a peptide is selected from a large library of phage-displayed peptides using an affinity purification method. Suitable purification methods include, *e.g.*, biopanning (Whaley *et al.,* 2000, Selection of peptides with semiconductor binding specificity for directed nanocrystal assembly, Nature 405(6787): 665-68) and affinity column chromatography. In each case, target quantum dots are immobilized and the recombinant phage display library is incubated against the immobilized quantum dots. Several rounds of biopanning are carried out and phage exhibiting affinity for the quantum dots are identified by standard methods after which the specificity of the peptides are tested using standard ELISA methodology.

Typically, the affinity purification is an iterative process that uses several affinity purification steps. Affinity purification may been used to identify peptides with affinity for particular metals and semiconductors (Belcher, 2001, Evolving Biomolecular Control of Semiconductor and Magnetic Nanostructure, presentation at Nanoscience: Underlying Physical Concepts and Properties, National Academy of Sciences, Washington, D.C., May 18-20, 2001; Belcher *et al.,* 2001, Abstracts of Papers, 222nd ACS National Meeting, Chicago, IL, United States, August 26-30,2001, American Chemical Society, Washington, D.C.).

An alternate method is directed toward the use of libraries of phage-displayed single chain variable domains, and to carry out the same type of selection process. Accordingly, in certain embodiments, a functional element is incorporated into a nanostructure through the use of joining elements (interaction sites) by which non-proteinaceous nanoparticles having desirable properties are attached to the nanostructure. Such joining elements are, in two non-limiting examples, derived from the complementarity determining regions of antibody variable domains or from affinity selected peptides.

Routine tests for electronic and photonic functional elements that are commonly used to compare the electronic properties of nanocrystals (single nanoparticles) and assemblies of nanoparticles (Murray *et al*., 2000, Synthesis and characterization of monodisperse nanocrystals and close-packed nanocrystal assemblies, Ann. Rev. Material Science 30: 545-610), are used for the analysis of nanostructures fabricated using the compositions and methods disclosed herein.

In certain embodiments, the unique, tunable properties of semiconductor nanocrystals make them preferable for use in nanodevices, including photoconductive nanodevices and light emitting diodes. The electrical properties of an individual nanostructure are difficult to measure, and therefore, photoconductivity is used as a measure of the properties of those nanostructures. Photoconductivity is a well-known phenomena used for analysis of the properties of semiconductors and organic solids. Photoconductivity has long been used to transport electrons between weakly interacting molecules in otherwise insulating organic solids.

Photocurrent spectral responses may also be used to map the absorption spectra of the nanocrystals in nanostructures and compared to the photocurrent spectral responses of individual nanocrystals (*see, e.g.,* Murray *et al*., 2000, Synthesis and characterization of monodisperse nanocrystals and close-packed nanocrystal assemblies, Ann. Rev. Material Science 30: 545-610). In addition, optical and photoluminescence spectra may also be used to study the optical properties of nanostructures (*see, e.g.,* Murray *et al*., 2000, Synthesis and characterization of monodisperse nanocrystals and close-packed nanocrystal assemblies, Ann. Rev. Material Science 30: 545-610).

The greater the control exerted over the formation of arrays of nanoparticles, the wider the array of optical, electrical and magnetic phenomena that will be produced. With staged assembly of nanostructures into which nanoparticles are incorporated with three-dimensional precision, it is possible to control the properties of solids formed therefrom in three dimensions, thereby giving rise to a host of anisotropic properties useful in the design of nanodevices. Routine tests and methods for characterizing the properties of these assemblages are well-known in the art (*see, e.g.,* Murray *et al*., 2000, Synthesis and characterization of monodisperse nanocrystals and close-packed nanocrystal assemblies, Ann. Rev. Material Sci. 30: 545-610).

For example, biosensors are commercially available that are made of a combination of proteins and quantum dots (Alivisatos *et al.,* 1996, Organization of 'nanocrystal molecules' using DNA, Nature 382: 609-11; Weiss *et al.,* U.S. Patent No. 6,207,392 entitled "Semiconductor nanocrystal probes for biological applications and process for making and using such probes," issued March 27, 2001). The ability to complex a quantum dot with a highly specific biological molecule (*e.g.*, a single stranded DNA or an antibody molecule) provides a spectral fingerprint for the target of the molecule. Using different sized quantum dots (each with very different spectral properties), each complexed to a molecule with different specificity, allows multiple sensing of components simultaneously.

Inorganic structures such as quantum dots and nanocrystals of metals or semiconductors may be used in the staged assembly of nanostructures as termini of branches of the assembled nanostructure. Once such inorganic structures are added, additional groups cannot be attached to them because they have an indeterminate stoichiometry for any set of binding sites engineered into a nanostructure. This influences the sequence in which assembly units are added to form a nanostructure being fabricated by staged assembly. For example, once a particular nanocrystal is added to the nanostructure, it is generally not preferred to add additional assembly units with joining elements that recognize and bind that type of nanocrystal, because it is generally not possible to control the positioning of such assembly units relative to the nanocrystal. Therefore, it may be necessary to add the nanocrystals last, or at least after all the assembly units that will bind that particular type of nanocrystal are added. In a preferred embodiment, nanocrystals are added to nanostructures that are still bound to a matrix and are sufficiently separated so that each nanocrystal can only bind to a single nanostructure, thereby preventing multiple cross-linking of nanostructures.

In one embodiment, a rigid nanostructure, fabricated according to the staged assembly methods of the present invention, comprises a magnetic nanoparticle attached as a functional element to the end of a nanostructure lever arm, which acts as a very sensitive sensor of local magnetic fields. The presence of a magnetic field acts to change the position of the magnetic nanoparticle, bending the nanostructure lever arm relative to the solid substrate to which it is attached. The position of the lever arm may be sensed, in certain embodiments, through a change in position of, for example, optical nanoparticles attached as functional elements to other positions (assembly units) along the nanostructure lever arm. The degree of movement of the lever arm is calibrated to provide a measure of the magnetic field.

In other embodiments, nanostructures that are fabricated according to the staged assembly methods of the invention have desirable properties in the absence of specialized functional elements. In such embodiments, a staged assembly process provides a two-dimensional or a three-dimensional nanostructure with small (nanometer-scale), precisely-sized, and well-defined pores that can be used, for example, for filtering particles in a microfluidic system. In further aspects of this embodiment, nanostructures are assembled that not only comprise such well-defined pores but also comprise functional elements that enhance the separation properties of the nanostructure, allowing separations based not only on size but also with respect to the charge and/or hydrophilicity or hydrophobicity properties of the molecules to be separated. Such nanostructures can be used for HPLC separations, providing extremely uniform packing materials and separations based upon those materials. Examples of such functional elements include, but are not limited to, peptide sequences comprising one or more side chains that are positively or negatively charged at a pH used for the desired chromatographic separation; and peptide sequences comprising one or more amino acids having hydrophobic or lipophilic side chains.

Junctions are architectural structures that can serve as "switch points" in microelectronic circuits such as silicon based electronic chips, etc. In certain embodiments, multivalent antibodies or binding derivatives or binding fragments thereof are used as junction structures and are introduced into nanostructures using the methods of the present invention. One non-limiting example of bioelectronic and biocomputational devices comprising these nanostructure junctions are quantum cellular automata (QCA).

### STAGED ASSEMBLY OF NANOSTRUCTURES

Pilin assembly units may be assembled to form nanostructures by staged assembly using, in one embodiment, the method disclosed in Section 6 (Example 1). This embodiment is also depicted in Fig. 3 and provides a schematic representation of the nanostructure intermediates formed.

Staged assembly enables massively parallel synthesis of complex, non-periodic, multi-dimensional nanostructures in which organic and inorganic moieties are placed, accurately and precisely, into a pre-designed, three-dimensional architecture. In a staged assembly, a series of assembly units is added in a given pre-designed order to an initiator unit and/or nanostructure intermediate. Because a large number of identical initiators are used and because subunits are added to all initiators/intermediates simultaneously, staged assembly fabricates multiple identical nanostructures in a massively parallel manner. In preferred embodiments, the initiator units are bound to a solid substrate, support or matrix. Additional assembly units are added sequentially in a procedure akin to solid phase polymer synthesis. The intermediate stage(s) of the nanostructure while it is being assembled, and which comprises the bound assembly units formed on the initiator unit, is generally described as either a nanostructure intermediate or simply, a nanostructure. Addition of each assembly unit to the nanostructure intermediate undergoing assembly depends upon the nature of the joining element presented by the previously added assembly unit and is independent of subsequently added assembly units. Thus assembly units can bind only to the joining elements exposed on the nanostructure intermediate undergoing assembly; that is, the added assembly units do not self-interact and/or polymerize.

Since the joining elements of a single assembly unit are non-complementary and therefore do not interact with one another, unbound assembly units do not form dimers or polymers. An assembly unit to be added is preferably provided in molar excess over the initiator unit or nanostructure intermediate in order to drive its reaction with the intermediate to completion. Removal of unbound assembly units during staged assembly is facilitated by carrying out staged assembly using a solid-substrate-bound initiator so that unbound assembly units can be washed away in each cycle of the assembly process.

This scheme provides for assembly of complex nanostructures using relatively few non-cross-reacting, complementary joining pairs. Only a few joining pairs need to be used, since only a limited number of joining elements will be exposed on the surface of an assembly intermediate at any one step in the assembly process. Assembly units with complementary joining elements can be added and incubated against the nanostructure intermediate, causing the added assembly units to be attached to the nanostructure intermediate during an assembly cycle. Excess assembly units can then be washed away to prevent them from forming unwanted interactions with other assembly units during subsequent steps of the assembly process. Each position in the nanostructure can be uniquely defined through the process of staged assembly and distinct functional elements can be added at any desired position. The staged assembly method of the invention enables massive parallel manufacture of complex nanostructures, and different complex nanostructures can be further self-assembled into higher order architectures in a hierarchic manner.

Fig. 4 depicts an embodiment of the staged assembly method of the invention in one dimension. In step 1, an initiator unit is immobilized on a solid substrate. In step 2, an assembly unit is added to the initiator (*i.e*. the matrix bound initiator unit), resulting in a nanostructure intermediate composed of two units. Only a single assembly unit is added in this step, because the second assembly unit cannot interact (*i.e.* polymerize) with itself.

The initiator unit, or any of the assembly units subsequently added during staged assembly including the capping unit, may contain an added functional element and/or may comprise a structural unit of different length from previously added units. For example, in step 3 of Fig. 4, a third assembly unit is added that comprises a functional element. In steps 4 and 5, additional assembly units are added, each with a designed functional group. Thus in the embodiment of staged assembly depicted in Fig. 4, the third, fourth and fifth assembly units each carry a unique functional element (designated by geometric shapes protruding from the top of the assembly units in the figure).

The embodiment of staged assembly depicted in Fig. 4 requires only two non-cross-reacting, complementary joining pairs. Self-assembly of the structure, as it stands at the end of step 5, would require four non-cross-reacting, complementary joining pairs. This relatively modest improvement in number of required joining pairs becomes far greater as the size of the structure increases. For instance, for a linear structure of N units assembled by an extension of the five steps illustrated in Fig. 4, staged assembly would still require only two non-cross-reacting, complementary joining pairs, whereas self-assembly would require (N-1) non-cross-reacting, complementary joining pairs.

The number of nanostructures fabricated is determined by the number of initiator units bound to the matrix while the length of each one-dimensional nanostructure is a function of the number of assembly cycles performed. If assembly units with one or more different functional elements are used, then the order of assembly will define the relative spatial orientation of each functional element relative to the other functional elements.

After each step in the method of staged assembly of the invention, excess unbound assembly units are removed from the attached nanostructure intermediate by a removal step, e.g., a washing step. The substrate-bound nanostructure intermediate may be washed with an appropriate solvent (*e.g.*, an aqueous solution or buffer). The solvent must be able to remove subunits held by non-specific interactions without disrupting the specific, interactions of complementary joining elements. Appropriate solvents may vary as to pH, salt concentration, chemical composition, etc., as required by the assembly units being used.

A buffer used for washing the nanostructure intermediate can be, for example, a buffer used in the wash steps implemented in ELISA protocols, such as those described in *Current Protocols in Immunology* (*see* Chapter 2, Antibody Detection and Preparation, Section 2.1 "Enzyme-Linked Immunosorbent Assays," John Wiley & Sons, 2001, Editors John E. Coligan, Ada M. Kruisbeek, David H. Margulies, Ethan M. Shevach, Warren Strober, Series Editor: Richard Coico).

In certain embodiments, an assembled nanostructure is "capped" by addition of a "capping unit," which is an assembly unit that carries only a single joining element. Furthermore, if the initiator unit has been attached to the solid substrate via a cleavable bond, the nanostructure can be removed from the solid substrate and isolated. However, in some embodiments, the completed nanodevice will be functional while attached to the solid substrate and need not be removed.

The above-described steps of adding assembly units can be repeated in an iterative manner until a complete nanostructure is assembled, after which time the complete nanostructure can be released by breaking the bond immobilizing the first assembly unit from the matrix at a designed releasing moiety (*e.g,*. a protease site) within the initiator unit or by using a pre-designed process for release (*e.g.*, lowering of pH). The process of staged assembly, as illustrated in FIGS. 2 and 3 is one of the simplest embodiments contemplated for staged assembly. In other embodiments, assembly units with additional joining elements can be used to create more complex assemblies. Assembly units may be added individually or, in certain embodiments, they can be added as subassemblies (Fig. 5). The result is a completely defined nanostructure with functional elements that are distributed spatially in relationship to one another to satisfy desired design parameters. The compositions and methods disclosed herein provide means for the assembly of these complex, designed nanostructures and of more complex nanodevices formed by the staged assembly of one or a plurality of nanostructures into a larger structure. Fabrication of multidimensional nanostructures can be accomplished, *e.g.*, by incorporating precisely-spaced assembly units containing additional joining elements into individual, one-dimensional nanostructures, where those additional joining elements can be recognized and bound by a suitable cross-linking agent to attach the individual nanostructures together. In certain preferred embodiments, such cross-linking could be, *e.g.*, an antibody or a binding derivative or a binding fragment thereof.

In some embodiments of the staged assembly method of the invention, the initiator unit is tethered to a solid support. Such tethering is not random (*i.e.*, is not non-specific binding of protein to plastic or random biotinylation of an assembly unit followed by binding to immobilized streptavidin) but involves the binding of a specific element of the initiator unit to the matrix or substrate. The staged assembly process is a vectorial process that requires an unobstructed joining element on the initiator unit for attachment of the next assembly unit. Random binding of initiator units to substrate would, in some cases, result in the obstruction of the joining element needed for the attachment of the next assembly unit, and thus lowering the number of initiator units on which nanostructures are assembled.

In other embodiments of the staged assembly method of the invention, the initiator unit is not immobilized to a solid substrate. In this case, a removal step, *e.g.*, a washing step, can be carried out on a nanostructure constructed on a non-immobilized or untethered initiator unit by: (1) attaching a magnetic nanoparticle to the initiator unit and separating nanostructure intermediates from non-bound assembly units by applying a magnetic field; 2) separating the larger nanostructure intermediates from unbound assembly units by centrifugation, precipitation or filtration; or 3) in those instances in which a nanostructure intermediate or assembled nanostructure is more resistant to a destructive treatment (*e.g.*, protease treatment or chemical degradation), unbound assembly units are selectively destroyed.

Proteins have well-defined binding properties, and the technology to manipulate the intermolecular interactions of proteins is well known in the art (Hayashi *et al*., 1995, A single expression system for the display, purification and conjugation of single-chain antibodies, Gene 160(1): 129-30; Hayden *et al.,* 1997, Antibody engineering, Curr. Opin. Immunol. 9(2): 201-12; Jung *et al.,* 1999, Selection for improved protein stability by phage display, J. Mol. Biol. 294(1): 163-80, Viti *et al.,* 2000, Design and use of phage display libraries for the selection of antibodies and enzymes, Methods Enzymol. 326: 480-505; Winter *et al.,* 1994, Making antibodies by phage display technology, Annu. Rev. Immunol. 12: 433-55). The contemplated staged assembly of nanostructures, however, need not be limited to components composed primarily of biological molecules, *e.g.*, proteins and nucleic acids, that have specific recognition properties. The optical, magnetic or electrical properties of inorganic atoms or molecules will be required for some embodiments of nanostructures fabricated by staged assembly.

There will be many embodiments of this invention in which components not made up of proteins will be advantageously utilized. In other embodiments, it may be possible to utilize the molecular interaction properties of proteins or nucleic acids to construct nanostructures composed of both organic and inorganic materials.

In certain embodiments, inorganic nanoparticles are added to components that are assembled into nanostructures using the staged assembly methods of the invention. This may be done using joining elements specifically selected for binding to inorganic particles. For example, Whaley and co-workers have identified peptides that bind specifically to semiconductor binding surfaces (Whaley *et al*., 2000, Selection of peptides with semiconductor binding specificity for directed nanocrystal assembly, Nature 405: 665-68). In one embodiment, these peptides are inserted into protein components described herein using standard cloning techniques. Staged assembly of protein constructs as disclosed herein, provides a means of distributing these binding sites in a rigid, well-defined three-dimensional array.

Once the binding sites for a particular type of inorganic nanoparticle are all in place, the inorganic nanoparticles can be added using a cycle of staged assembly analogous to that used to add proteinaceous assembly units. To accomplish this, it may be necessary, in certain embodiments to adjust the solution conditions under which the nanostructure intermediates are incubated, in order to provide for the solubility of the inorganic nanoparticles. Once an inorganic nanoparticle is added to the nanostructure intermediate, it is not possible to add further units to the inorganic nanoparticle in a controlled fashion because of the microheterogeneities intrinsic to any population of inorganic nanoparticles. These heterogeneities would render the geometry and stoichiometry of further interactions uncontrollable.

Fig. 6 is a diagram illustrating the addition of protein units and inorganic elements to a nanostructure according to the staged assembly methods of the invention. In step 1, an initiator unit is bound to a solid substrate. In step 2, an assembly unit is bound specifically to the initiator unit. In step 3, an additional assembly unit is bound to the nanostructure undergoing assembly. This assembly unit comprises an engineered binding site specific for a particular inorganic element. In step 4, the inorganic element (depicted as a cross-hatched oval) is added to the structure and bound by the engineered binding site. Step 5 adds another assembly unit with a binding site engineered for specificity to a second type of inorganic element, and that second inorganic element (depicted as a hatched diamond) is added in step 6.

The order in which assembly units are added is determined by the desired structure and/or activity that the product nanostructure, and the need to minimize the number of cross-reacting joining element pairs used in the assembly process. Hence determining the order of assembly is an integral part of the design of a nanostructure to be fabricated by staged assembly. Joining elements are chosen, by design, to permit staged assembly of the desired nanostructure. Since the choice of joining element(s) is generally independent of the functional elements to be incorporated into the nanostructure, the joining elements are mixed and matched as needed to fabricate assembly units with the necessary functional elements and joining elements that will provide for the placement of those functional elements in the desired spatial orientation.

For example, assembly units comprising two joining elements, designed using the six joining elements that make up three joining pairs, can include any of 18 pairs of the joining elements that are non-interacting. There are 21 possible pairs of joining elements, but three of these pairs are interacting (e.g. A-A') and their use in an assembly unit would lead to the self-association of identical assembly units with one another. In the example illustrated below, joining elements are denoted as A, A', B, B', C and C', where A and A', B and B', and C and C' are complementary pairs of joining elements (joining pairs), *i.e.* they bind to each other with specificity, but not to any of the other four joining elements depicted. Six representative assembly units, each of which comprises two joining elements, wherein each joining element comprises a non-identical, non-complementary joining element, are depicted below. In this depiction, each assembly unit further comprises a unique functional element, one of a set of six, and represented as F₁ to F₆. According to these conventions, six possible assembly units can be designated as:

A-F₁-B

B'-F₂-A'

B'-F₃-C'

C-F₄-B

B'-F₅-A'

A-F₆-C'

Staged assembly according to the methods disclosed herein can be used to assemble the following illustrative linear, one-dimensional nanostructures, in which the order and relative vectorial orientation of each assembly unit is independent of the order of the functional elements (the symbol ●- is used to represent the solid substrate to which the initiator is attached and a double colon represents the specific interaction between assembly units):
●-A-F1-B::B'-F2-A'::A-F1-B::B'-F2-A'::A-F1-B::B'-F2-A'::A-F1-B::B'-F2-A
●-A-F1-B::B'-F2-A'::A-F6-C'::C-F4-B::B'-F2-A'::A-F1-B::B'-F5-A'::A-F6-C'
●-A-F1-B::B'-F2-A'::A-F1-B::B'-F5-A'::A-F1-B::B'-F2-A'::A-F1-B::B'-F3-C'
●-A-F1-B::B'-F3-C'::C-F4-B::B'-F3-C'::C-F4-B::B'-F3-C::C-F4-B::B'-F2-A'

As is apparent from this illustration, a large number of unique assembly units can be constructed using a small number of complementary joining elements. Moreover, only a small number of complementary joining elements are required for the fabrication of a large number of unique and complex nanostructures, since only one type of assembly unit is added in each staged assembly cycle and, therefore, joining elements can be used repeatedly without rendering ambiguous the position of an assembly unit within the completed nanostructure.

In each of the cases illustrated above, only two or three joining pairs have been used. Self-assembly of any of these structures would require the use of seven non-cross-reacting joining pairs. If these linear structures were N units in extent and assembled using staged assembly, they would still only require two or three joining pairs, but for self-assembly, they would require (N-1) non-cross-reacting, complementary joining pairs.

In another aspect of the invention, by interchanging the positions of the two joining elements of an assembly unit depicted above, the spatial position and orientation of the attached functional element will be altered within the overall structure of the nanostructure fabricated. This aspect of the invention illustrates yet another aspect of the design flexibility provided by staged assembly of nanostructures as disclosed herein.

Attachment of each assembly unit to an initiator or nanostructure intermediate is mediated by formation of a specific joining-pair interaction between one joining element of the assembly unit and one or more unbound complementary joining elements carried by the initiator or nanostructure intermediate. In many embodiments, only a single unbound complementary joining element will be present on the initiator or nanostructure intermediate. However, in other embodiments, it may be advantageous to add multiple identical assembly units to multiple sites on the assembly intermediate that comprise identical joining elements. In these embodiments, the staged assembly proceeds by the parallel addition of assembly units, but only a single unit will be attached at any one site on the intermediate, and assembly at all sites that are involved will occur in a pre-designed, vectorial manner.

Structural integrity of the nanostructure is of critical importance throughout the process of staged assembly, and the assembly units are preferably connected by non-covalent interactions. A specific non-covalent interaction is, for example, an interaction that occurs between an assembly unit and a nanostructure intermediate. The specific interaction should exhibit adequate affinity to confer stability to the complex between the assembly unit and the nanostructure intermediate sufficient to maintain the interaction stably throughout the entire staged assembly process. A specific non-covalent interaction should exhibit adequate specificity such that the added assembly unit will form stable interactions only with joining elements designed to interact with it. The interactions that occur among elements during the staged assembly process disclosed herein are preferably operationally "irreversible." A binding constant that meets this requirement cannot be defined unambiguously since "irreversible" is a kinetic concept, and a binding constant is based on equilibrium properties. Nevertheless, interactions with Kd's of the order of 10⁻⁷ or lower (*i.e.* higher affinity and similar to the Kd of a typical diabody-epitope complex) will typically act "irreversibly" on the time scale of interest, *i.e.* during staged assembly of a nanostructure.

The intermolecular interactions need not act "irreversibly," however, on the timescale of the utilization of a nanostructure (*i.e.* its shelf life or working life expectancy). In certain embodiments, nanostructures fabricated according to the staged assembly methods disclosed herein are subsequently stabilized by chemical fixation (*e.g.*, by fixation with paraformaldehyde or glutaraldehyde) or by cross-linking. The most common schemes for cross-linking two proteins involve the indirect coupling of an amine group on one assembly unit to a thiol group on a second assembly unit (*see, e.g.,* Handbook of Fluorescent Probes and Research Products, Eighth Edition, Chapter 2, Molecular Probes, Inc., Eugene, OR; Loster *et al.,* 1997, Analysis of protein aggregates by combination of cross-linking reactions and chromatographic separations, J. Chromatogr. B. Biomed. Sci. Appl. 699(1-2): 439-61; Phizicky *et al.,* 1995, Protein-protein interactions: methods for detection and analysis, Microbiol. Rev. 59(1): 94-123).

In certain embodiments of the invention, the fabrication of a nanostructure by the staged assembly methods of the present invention involves joining relatively rigid and stable assembly units, using non-covalent interactions between and among assembly units. Nevertheless, the joining elements that are incorporated into useful assembly units can be rather disordered, that is, neither stable nor rigid, prior to interaction with a second joining element to form a stable, preferably rigid, joining pair. Therefore, in certain embodiments of the invention, individual assembly units may include unstable, flexible domains prior to assembly, which, after assembly, will be more rigid. In preferred embodiments, a nanostructure fabricated using the compositions and methods disclosed herein is a rigid structure.

According to the methods of the invention, analysis of the rigidity of a nanostructure, as well as the identification of any architectural flaws or defects, are carried out using methods well-known in the art, such as electron microscopy.

In another embodiment, structural rigidity can be tested by attaching one end of a completed nanostructure directly to a solid surface, *i.e.*, without the use of a flexible tether. The other end of the nanostructure (or a terminal branch of the nanostructure, if it is a multibranched structure) is then attached to an atomic force microscope (AFM) tip, which is movable. Force is applied to the tip in an attempt to move it. If the nanostructure is flexible, there will be an approximately proportional relationship between the force applied and tip movement as allowed by deflection of the nanostructure. In contrast, if the nanostructure is rigid, there will be little or no deflection of the nanostructure and tip movement as the level of applied force increases, up until the point at which the rigid nanostructure breaks. At that point, there will be a large movement of the AFM tip even though no further force is applied. As long as the attachment points of the two ends are stronger than the nanostructure, this method will provide a useful measurement of rigidity.

According to the present invention, each position in a nanostructure is distinguishable from all others, since each assembly unit can be designed to interact tightly, specifically, and uniquely with its neighbors. Each assembly unit can have an activity and/or characteristic that is distinct to its position within the nanostructure. Each position in the nanostructure is uniquely defined through the process of staged assembly, and through the properties of each assembly unit and/or functional element that is added at a desired position. In addition, the staged-assembly methods and assembly units disclosed herein are amenable to large scale, massively parallel, automated manufacturing processes for construction of complex nanostructures of well-defined size, shape, and function.

The methods and compositions of the present invention capitalize upon the precise dimensions, uniformity and diversity of spatial geometries that proteins are capable of that are used in the construction of the assembly units employed herein. Furthermore, as described hereinbelow, the methods of the invention are advantageous because genetic engineering techniques can be used to modify and tailor the properties of those biological materials used in the methods of the invention disclosed herein, as well as to synthesize large quantities of such materials in microorganisms.

The present invention also provides for the staged assembly of nanostructures that utilizes assembly units comprising a fragment of a protein of interest, *e.g.*, an antibody or pilin protein. In a specific embodiment of the invention, a protein consisting of or comprising a fragment of a protein of interest consists of at least 4 contiguous amino acids of the protein of interest. In other embodiments, the fragment consists of at least 5, 6, 7, 8, 9, 10, 15, 20, 35 or 50 contiguous amino acids of the protein of interest. In specific embodiments, such fragments are not larger than 35, 100, 200, 300 or 350 amino acids.

The present invention also provides for the staged assembly of nanostructures that utilizes assembly units comprising fusion proteins. The production of fusion or chimeric protein products (comprising a desired protein (*e.g.*, an IgG), fragment, analog, or derivative joined via a peptide bond to a heterologous protein sequence (of a different protein)). Such chimeric protein products can be made by ligating the appropriate nucleic acid sequences encoding the desired amino acid sequences to each other by methods known in the art, in the proper reading frame, and expressing the chimeric product by methods commonly known in the art. Alternatively, such a chimeric product may be made by protein synthetic techniques, *e.g.*, by use of a peptide synthesizer.

### Initiator Assembly Units

An initiator assembly unit is the first assembly unit incorporated into a nanostructure that is formed by the staged assembly method of the invention. An initiator assembly unit may be attached, in certain embodiments, by covalent or non-covalent interactions, to a solid substrate or other matrix. An initiator assembly unit is also known as an "initiator unit."

Staged assembly of a nanostructure begins by the non-covalent, vectorial addition of a selected assembly unit to the initiator unit. According to the methods of the invention, an assembly unit is added to the initiator unit through (i) the incubation of an initiator unit, which in some embodiments, is immobilized to a matrix or substrate, in a solution comprising the next assembly unit to be added. This incubation step is followed by (ii) a removal step, *e.g.*, a washing step, in which excess assembly units are removed from the proximity of the initiator unit.

Assembly units bind to the initiator unit through the formation of specific, non-covalent bonds. The joining elements of the next assembly unit are chosen so that they attach only at pre-designated sites on the initiator unit. Only one assembly unit can be added to a target joining element on the initiator unit during the first staged-assembly cycle, and binding of the assembly unit to the target initiator unit is vectorial. Staged assembly continues by repeating steps (i) and (ii) until all of the desired assembly units are incorporated into the nanostructure according to the desired design of the nanostructure.

In a preferred embodiment of the staged assembly method of the invention, an initiator unit is immobilized on a substrate and additional units are added sequentially in a procedure analogous to solid phase polymer synthesis.

An initiator unit is a category of assembly unit, and therefore can comprise any of the structural, joining, and/or functional elements described hereinbelow as being comprised in an assembly unit of the invention. An initiator unit can therefore comprise any of the following molecules, or a binding derivative or binding fragment thereof: a monoclonal antibody; a multispecific antibody, a Fab or F(ab')₂ fragment, a single-chain antibody fragment (scFv); a bispecific, chimeric or bispecific heterodimeric F(ab')₂; a diabody or multimeric scFv fragment; a bacterial pilin protein, a leucine zipper-type coiled coil, a four-helix bundle, a peptide epitope, or a PNA, or any other type of assembly unit disclosed herein.

In certain embodiments, the invention provides an initiator assembly unit which comprises at least one joining element. In other embodiments, the invention provides an initiator assembly unit with two or more joining elements.

Initiator units may be tethered to a matrix in a variety of ways. The choice of tethering method will be determined by several design factors including, but not limited to: the type of initiator unit, whether the finished nanostructure must be removed from the matrix, the chemistry of the finished nanostructure, etc. Potential tethering methods include, but are not limited to, antibody binding to initiator epitopes, His tagged initiators, initiator units containing matrix binding domains (*e.g.*, chitin-binding domain, cellulose-binding domain), antibody binding proteins (*e.g.*, protein A or protein G) for antibody or antibody-derived initiator units, streptavidin binding of biotinylated initiators, PNA tethers, and specific covalent attachment of initiators to matrix.

In certain embodiments, an initiator unit is immobilized on a solid substrate. Initiator units may be immobilized on solid substrates using methods commonly used in the art for immobilization of antibodies or antigens. There are numerous methods well known in the art for immobilization of antibodies or antigens. These methods include non-specific adsorption onto plastic ELISA plates; biotinylation of a protein, followed by immobilization by binding onto streptavidin or avidin that has been previously adsorbed to a plastic substrate (*see, e.g.,* Sparks *et al.,* 1996, Screening phage-displayed random peptide libraries, in Phage Display of Peptides and Proteins, A Laboratory manual, editors, B.K. Kay, J. Winter and J. McCafferty, Academic Press, San Diego, pp. 227-53). In addition to ELISA microtiter plates, protein may be immobilized onto any number of other solid supports such as Sepharose (Dedman *et al.,* 1993, Selection of target biological modifiers from a bacteriophage library of random peptides: the identification of novel calmodulin regulatory peptides, J. Biol. Chem. 268; 23025-30) or paramagnetic beads (Sparks *et al*., 1996, Screening phage-displayed random peptide libraries, in Phage Display of Peptides and Proteins, A Laboratory manual, editors, B.K. Kay, J. Winter and J. McCafferty, Academic Press, San Diego, pp. 227-53). Additional methods that may be used include immobilization by reductive amination of amine-containing biological molecules onto aldehyde-containing solid supports (Hermanson, 1996, Bioconjugate Techniques, Academic Press, San Diego, p. 186), and the use of dimethyl pimelimidate (DMP), a homobifunctional cross-linking agent that has imidoester groups on either end (Hermanson, 1996, Bioconjugate Techniques, Academic Press, San Diego, pp. 205-06). This reagent has found use in the immobilization of antibody molecules to insoluble supports containing bound protein A (*e.g.*, Schneider *et al.,* 1982, A one-step purification of membrane proteins using a high efficiency immunomatrix, J. Biol. Chem. 257, 10766-69).

In a specific embodiment, an initiator unit is a diabody that comprises a tethering domain (T) that recognizes and binds an immobilized antigen/hapten and an opposing domain (A) to which additional assembly units are sequentially added in a staged assembly. Antibody 8F5, which is directed against the antigenic peptide VKAETRLNPDLQPTE (SEQ ID NO: 13) derived human rhinovirus (Serotype 2) viral capsid protein Vp2, is used as the T domain (Tormo *et al.,* 1994, Crystal structure of a human rhinovirus neutralizing antibody complexed with a peptide derived from viral capsid protein VP2, EMBO J. 13(10): 2247-56). The A domain is the same lysozyme anti-idiotopic antibody (E5.2) previously described for Diabody Unit 1. The completed initiator assembly unit therefore contains 8F5 x 730.1.4 (T x A) as the opposing CDRs. The initiator unit is constructed and functionally characterized using the methods described herein for characterizing joining elements and/or structural elements comprising diabodies.

In order to immobilize the initiator unit onto a solid support matrix, the rhinovirus antigenic peptide may fused to the protease recognition peptide factor Xa through a short flexible linker spliced at the N termini of the Factor Xa sequence, IEGR, (Nagai and Thogersen, 1984, Generation of beta-globin by sequence-specific proteolysis of a hybrid protein produced in Escherichia coli, Nature309(5971): 810-12) and between the Factor Xa sequence and the antigenic peptide sequence. This fusion peptide may be covalently linked to CH-Sepharose 4B (Pharmacia); a sepharose derivative that has a six-carbon long spacer arm and permits coupling *via* primary amines. (Alternatively, Sepharose derivatives for covalent attachment via carboxyl groups may be used.) The covalently attached fusion protein will serve as a recognition epitope for the tethering domain "8F5" in the initiator unit (T x A).

Once the initiator is immobilized, additional diabody units (diabody assembly units 1 and 2) may be sequentially added in a staged assembly, unidirectionally from binding domain A'. Upon completion of the staged assembly, the nanostructure may be either cross-linked to the support matrix or released from the matrix upon addition of the protease Factor Xa. The protease will cleave the covalently attached antigenic /Factor Xa fusion peptide, releasing the intact nanostructure from the support matrix, since, by design, there are no Factor Xa recognition sites contained within any of the designed protein assembly units.

An alternate strategy of cleaving the peptide fusion from the solid support matrix that does not require the addition of Factor Xa, can also be implemented. This method utilizes a cleavable spacer arm attached to the sepharose matrix. The antigen peptide is covalently attached through a phenyl-ester linkage to the matrix. Once the immobilized antibody binds initiator assembly unit, the initiator assembly unit remains tethered to the support matrix until chemical cleavage of the spacer arm with imidazoleglycine buffer at pH 7.4 at which point the initiator unit/antigen complex (and associated nanostructure) are released from the support matrix.

### METHODS FOR CHARACTERIZING JOINING ELEMENTS

### METHODS FOR CHARACTERIZING JOINING- ELEMENT INTERACTIONS USING X-RAY CRYSTALLOGRAPHY

In many instances, molecular recognition between proteins or between proteins and peptides may be determined experimentally. In one aspect of the invention, the protein-protein interactions that define the joining element interactions, and are critical for formation of a joining pair are characterized and identified by X-ray crystallographic methods commonly known in the art. Such characterization enables the skilled artisan to recognize joining pair interactions that may be useful in the compositions and methods of the present invention.

### METHODS FOR CHARACTERIZING JOINING- ELEMENT SPECIFICITY AND AFFINITY

Verification that two complementary joining elements interact with specificity may be established using, for example, ELISA assays, analytical ultracentrifugation, or BIAcore methodologies (Abraham *et al.,* 1996, Determination of binding constants of diabodies directed against prostate-specific antigen using electrochemiluminescence-based immunoassays, J. Mol. Recognit. 9(5-6): 456-61; Atwell *et al.,* 1996, Design and expression of a stable bispecific scFv dimer with affinity for both glycophorin and N9 neuraminidase, Mol. Immunol. 33(17-18): 1301-12; Muller *et al.* 1998), A dimeric bispecific miniantibody combines two specificities with avidity, FEBS Lett. 432(1-2): 45-49), or other analogous methods well known in the art, that are suitable for demonstrating and/or quantitating the strength of intermolecular binding interactions.

### DESIGN AND ENGINEERING OF STRUCTURAL, JOINING AND FUNCTIONAL ELEMENTS

Design of structural, joining and functional elements of the invention, and of the assembly units that comprise them, is facilitated by analysis and determination of those amino acid residues in the desired binding interaction, as revealed in a defined crystal structure, or through homology modeling based on a known crystal structure of a highly homologous protein. The crystal structure of, *e.g.*, a pilin-peptide complex, may be used to predict the structure and geometry of pilin-pilin interactions. Although a complex between two pilin proteins has yet to be crystallized, energy calculations and solid-body modeling can be used to predict the structure of a complex made up of multiple pilins (Sauer et al., 1999, Structural basis of chaperone function and pilus biogenesis; Science 285: 1058-1061; Choudhury et al., 1999, X-ray structure of the FimC-FimH chaperone-adhesin complex from uropathogenic Escherichia coli, Science 285: 1061-1066).

Design of a useful assembly unit comprising one or more functional elements preferably involves a series of decisions and analyses that may include, but are not limited to, some or all of the following steps:
(i) selection of the functional elements to be incorporated based on the desired overall function of the nanostructure;
(ii) selection of the desired geometry based on the target function, in particular, determination of the relative positions of the functional elements;
(iii)selection of joining elements through determination, identification or selection of those peptides or proteins, *e.g.* from a combinatorial library, that have specificity for the functional nanoparticles to be incorporated into the desired nanostructure;
(iv) based on the needed separations between functional elements comprising, e.g. nanoparticles such as quantum dots, etc., selection of structural elements that will provide a suitably rigid structure with correct dimensions and having positions for incorporation of joining elements with the correct geometry and stoichiometry;
(v)design of fusion proteins incorporating peptide or protein joining elements, from step (iii) and the structural element selected in step (iv) such that the folding of the structural and joining elements of the assembly unit are not disrupted (*e.g.*, through incorporation at β-turns);
(vi) computer modeling of the resultant fusion proteins in the context of the overall design of the nanostructure and refining of the design to optimize the structural dimensions as required by the functional specifications; or
(vii) design of the assembly sequence for staged assembly.

Modification of a structural element protein, for example, usually involves insertion, deletion, or modification of the amino acid sequence of the protein in question. In many instances, modifications involve insertions or substitutions to add joining elements not extant in the native protein. A non-limiting example of a routine test to determine the success of an insertion mutation is a circular dichroism (CD) spectrum. The CD spectrum of the resultant fusion mutant protein can be compared to the CD of the native protein.

If the insert is small (*e.g.*, a short peptide), then the spectra of a properly folded insertion mutant will be very similar to the spectra of the native protein. If the insertion is an entire protein domain (*e.g.* single chain variable domain), then the CD spectrum of the fusion protein should correspond to the sum of the CD spectra of the individual components (*i.e.* that of the native protein and fusion protein comprising the native protein and the functional element). This correspondence provides a routine test for the correct folding of the two components of the fusion protein.

Preferably, a further test of the successful engineering of a fusion protein is made. For example, an analysis may be made of the ability of the fusion protein to bind to all of its targets, and therefore, to interact successfully with all joining pairs. This may be performed using a number of appropriate ELISA assays; at least one ELISA is performed to test the affinity and specificity of the modified protein for each of the joining pairs required to form the nanostructure.

### USES OF THE STAGED-ASSEMBLY METHOD AND OF NANOSTRUCTURES CONSTRUCTED THEREBY

The staged-assembly methods and the assembly units of the invention have use in the construction of myriad nanostructures. The uses of such nanostructures are readily apparent and include applications that require highly regular, well-defined arrays of one-, two-, and three-dimensional structures such as fibers, cages, or solids, which may include specific attachment sites that allow them to associate with other materials.

In certain embodiments, the nanostructures fabricated by the staged assembly methods of the invention are one-dimensional structures. For example, nanostructures fabricated by staged assembly can be used for structural reinforcement of other materials, *e.g.*, aerogels, paper, plastics, cement, etc. In certain embodiments, nanostructures that are fabricated by staged assembly to take the form of long, one-dimensional fibers are incorporated, for example, into paper, cement or plastic during manufacture to provide added wet and dry tensile strength.

In another embodiment, the nanostructure is a patterned or marked fiber that can be used for identification or recognition purposes. In such embodiments, the nanostructure may contain such functional elements as *e.g.*, a fluorescent dye, a quantum dot, or an enzyme.

In a further embodiment, a particular nanostructure is impregnated into paper and fabric as an anti-counterfeiting marker. In this case, a simple color-linked antibody reaction (such as those commercially available in kits) is used to verify the origin of the material. Alternatively, such a nanostructure could bind dyes, inks or other substances, either before or after incorporation, to color the paper or fabrics or to modify their appearance or properties in other ways.

In another embodiment, nanostructures are incorporated, for example, into ink or dyes during manufacture to increase solubility or miscibility.

In another embodiment, a one-dimensional nanostructure *e.g.*, a fiber, bears one or more enzyme or catalyst functional elements in desired positions. The nanostructure serves as a support structure or scaffold for an enzymatic or catalytic reaction to increase its efficiency. In such an embodiment, the nanostructure may be used to "mount" or position enzymes or other catalysts in a desired reaction order to provide a reaction "assembly line."

In another embodiment, a one-dimensional nanostructure, *e.g.*, a fiber, is used as an assembly jig. Two or more components, *e.g.*, functional units, are bound to the nanostructure, thereby providing spatial orientation. The components are joined or fused, and then the resultant fused product is released from the nanostructure.

In another embodiment, a nanostructure is a one-, two- or three-dimensional structure that is used as a support or framework for mounting nanoparticles (*e.g.*, metallic or other particles with thermal; electronic or magnetic properties) with defined spacing, and is used to construct a nanowire or nanocircuit.

In another embodiment, the staged assembly methods of the invention are used to accomplish electrode-less plating of a one-dimensional nanostructure (fiber) with metal to construct a nanowire with a defined size and/or shape. For example, a nanostructure could be constructed that comprises metallic particles as functional elements.

In another embodiment, a one-dimensional nanostructure (*e.g.*, a fiber) comprising magnetic particles as functional elements is aligned by an external magnetic field to control fluid flow past the nanostructure. In another embodiment, the external magnetic field is used to align or dealign a nanostructure (*e.g.*, fiber) comprising optical moieties as functional elements for use in LCD-type displays.

In another embodiment, a nanostructure is used as a size standard or marker of precise dimensions for electron microscopy.

In other embodiments, the nanostructures fabricated by the staged assembly methods of the invention are two- or three-dimensional structures. For example, in one embodiment, the nanostructure is a mesh with defined pore size and can serve as a two-dimensional sieve or filter.

In another embodiment, the nanostructure is a three-dimensional hexagonal array of assembly units that is employed as a molecular sieve or filter, providing regular vertical pores of precise diameter for selective separation of particles by size. Such filters can be used for sterilization of solutions (*i.e.*, to remove microorganisms or viruses), or as a series of molecular-weight cut-off filters. In this embodiment, the protein components of the pores, such as structural elements or functional elements, may be modified so as to provide specific surface properties (*i.e.*, hydrophilicity or hydrophobicity, ability to bind specific ligands, etc.). Among the advantages of this type of filtration device is the uniformity and linearity of pores and the high pore to matrix ratio.

It will be apparent to one skilled in the art that the methods and assembly units disclosed herein may be used to construct a variety of two- and three-dimensional structures such as polygonal structures (*e.g.*, octagons), as well as open solids such as tetrahedrons, icosahedrons formed from triangles, and boxes or cubes formed from squares and rectangles (*e.g.*, the cube disclosed in Section 11, Example 6). The range of structures is limited only by the types of joining and functional elements that can be engineered on the different axes of the structural elements.

In another embodiment, a two-or three-dimensional nanostructure may be used to construct a surface coating comprising optical, electric, magnetic, catalytic, or enzymatic moieties as functional units. Such a coating could be used, for example, as an optical coating. Such an optical coating could be used to alter the absorptive or reflective properties of the material coated.

A surface coating constructed using nanostructures of the invention could also be used as an electrical coating, *e.g.*, as a static shielding or a self-dusting surfaces for a lens (if the coating were optically clear). It could also be used as a magnetic coating, such as the coating on the surface of a computer hard drive.

Such a surface coating could also be used as a catalytic or enzymatic coating, for example, as surface protection. In a specific embodiment, the coating is an antioxidant coating.

In another embodiment, the nanostructure may be used to construct an open framework or scaffold with optical, electric, magnetic, catalytic, enzymatic moieties as functional elements. Such a scaffold may be used as a support for optical, electric, magnetic, catalytic, or enzymatic moieties as described above. In certain embodiments, such a scaffold could comprise functional elements that are arrayed to form thicker or denser coatings of molecules, or to support soluble micron-sized particles with desired optical, electric, magnetic, catalytic, or enzymatic properties.

In another embodiments, a nanostructure serves as a framework or scaffold upon which enzymatic or antibody binding domains could be linked to provide high density multivalent processing sites to link to and solubilize otherwise insoluble enzymes, or to entrap, protect and deliver a variety of molecular species.

In another embodiment, the nanostructure may be used to construct a high density computer memory with addressable locations.

In another embodiment, the nanostructure may be used to construct an artificial zeolite, *i.e.*, a natural mineral (hydrous silicate) that has the capacity to absorb ions from water, wherein the design of the nanostructure promotes high efficiency processing with reactant flow-through an open framework.

In another embodiment, the nanostructure may be used to construct an open framework or scaffold that serves as the basis for a new material, *e.g.*, the framework may possess a unique congruency of properties such as strength, density, determinate particle packing and/or stability in various environments.

Inc certain embodiments, the staged-assembly methods of the invention can also be used for constructing computational architectures, such as quantum cellular automata (QCA) that are composed of spatially organized arrays of quantum dots. In QCA technology, the logic states are encoded by positions of individual electrons, contained in QCA cells composed of spatially positioned quantum dots, rather than by voltage levels. Staged assembly can be implemented in an order that spatially organizes quantum dot particles in accordance with the geometries necessary for the storage of binary information. Examples of logic devices that can be fabricated using staged assembly for the spatially positioning and construction of QCA cells for quantum dot cellular automata include QCA wires, QCA inverters, majority gates and full adders (Amlani *et al*., 1999, Digital logic gate using quantum-dot cellular automata, Science 284(5412): 289-91; Cowburn and Welland, 2000), Room temperature magnetic quantum cellular automata, Science 287(5457): 1466-68; Orlov *et al*., 1997, Realization of a Functional Cell for Quantum-Dot Automata, Science 277: 928-32).

The invention will now be further described with reference to the following, non-limiting examples.

### EXAMPLE 1: STAGED ASSEMBLY OF HYBRID PILIN ASSEMBLY UNITS

In this example, hybrid pilin assembly units are constructed using the following steps of the staged-assembly methods of the invention.

With the immobilized papA and the hybrid proteins engineered as disclosed above, it is possible to assemble a filament comprising five pilin units and having two ras epitopes positioned, one each, on the second and fifth units in the assembly (Fig. 3).
(1) In the first step, PapA units are immobilized on a solid matrix using methods well known in the art. For example, a biotin moiety may be added to the amino terminus of papA; the papA then incubated in the presence of a surface coated with streptavidin. The very strong interaction of biotin with streptavidin will lead to the immobilization of papA on the surface. Many other methods for the immobilization of a protein on a solid surface are available and well known to those of ordinary skill in the art.
(2) In the second step, a solution of papH-papK hybrid protein displaying the ras epitope is incubated with the immobilized papA. In order to solubilize the papH-papK hybrid, it may be necessary to complex it with the chaperone papD. During incubation, papD will exchange with the immobilized papA to deposit the hybrid protein onto papA. After an appropriate incubation period, generally from seconds to minutes, in most cases, and upwards to several hours in unusual cases, any excess protein is washed off. The product of this step will be a pilin dimer comprising the immobilized papA and the hybrid papH-papK with ras epitope.
(3) In the third step, a solution of papE-papA hybrid protein (possibly in complex with papD) is incubated with the immobilized product of Step 2. After incubation any excess protein is washed off. The result of this step will be a pilin trimer comprising the immobilized papA, the hybrid papH-papK with ras epitope and the hybrid papE-papA protein (Fig. 3).
(4) In the fourth step, a solution of papK-papF hybrid protein is incubated, as described above in Step 3, with the immobilized product of Step 3. After incubation, any excess protein is washed off. This step produces a pilin tetramer comprising the immobilized papA, the hybrid papH-papK with ras epitope, the hybrid papE-papA protein and the hybrid papK-papF protein (Fig. 3).
(5) In the fifth step, a solution of papH-papE hybrid protein (possibly in complex with papD) with inserted ras epitope is incubated the immobilized product of Step 4. After incubation, any excess protein is washed off. The result of this step will be a pilin pentamer comprising the immobilized papA, the hybrid papH-papK with ras epitope, the hybrid papE-papA protein, the hybrid papK-papF protein and the papH-papE hybrid with ras epitope (FIG. 17).

It is possible to construct more complex structures through the continued addition of pilin units in a manner analogous to that used in steps 2-5.

### EXAMPLE 2: PROTOCOL FOR STAGED ASSEMBLY USING MULTISPECIFIC PROTEIN ASSEMBLY UNITS

The following steps of staged assembly are illustrated in Fig. 3. The resultant nanostructure is illustrated Fig. 3, Step 11.

| Staged Assembly Steps | Procedure |
|---|---|
| Step 1 | a) Add assembly unit-1 |
| | b) Wash |
| | |
| Step 2 | a) Add assembly unit-2 |
| | b) Wash |
| | |
| Step 3 | a) Repeat Step 1 |
| | |
| Step 4 | a) Add assembly unit-3 |
| | b) Wash |
| | |
| Step 5 | a) Repeat Step 1 |
| | |
| Step 6 | a) Add assembly unit-4 |
| | b) Wash |
| | |
| Step 7 | a) Repeat Step 2 |
| | |
| Step 8 | a) Add assembly unit-5 |
| | b) Wash |
| | |
| Step 9 | a) Repeat Step 1 |
| Step 10 | a) Repeat Step 2 |
| | |
| Step 11 | a) Repeat Step 1 |

### EXAMPLE 3: FABRICATION OF A MACROMOLECULAR NANOSTRUCTURE

To build a macromolecular assembly, two assembled nanostructures intermediates can be joined to one another using the staged assembly methods of the invention. This example describes the fabrication of a macromolecular nanostructure from two nanostructure intermediates.

Fig. 8 illustrates the staged assembly of the two nanostructure intermediates fabricated from the staged assembly protocol illustrated in Fig. 3. Nanostructure intermediate-1 is illustrated as Step-11 in Fig. 3. Nanostructure intermediate-2 is illustrated as Step-8 in Fig. 3. The following protocol describes the addition of two nanostructure intermediates by the association of a complementary joining pair as illustrated in Fig. 8. The resultant macromolecular nanostructure is illustrated Fig. 8, Step 5.

| Staged Assembly Steps | Procedure |
|---|---|
| Step 1 | Steps 1-11 of staged assembly protocol |
| | described above in Section 8 (Example 3) |
| | |
| | a) Add A' capping unit |
| Step 2 | b) Wash |
| | |
| Step 3 | Remove nanostructure intermediate-1 from |
| | the support matrix and isolate |
| | |
| Step 4 | Perform Steps 1-8 of staged assembly |
| | protocol described above in Section 8 |
| | (Example 3), leaving nanostructure |
| | intermediate-2 attached to the support |
| | matrix |
| | |
| Step 5 | a) Add nanostructure intermediate-1 |
| | b) Wash |

### EXAMPLE 4: ANALYSIS OF POLYMERIZATION BY LIGHT SCATTERING

The extent polymerization of assembly units may be analyzed by light scattering. Light scattering measurements from a light scattering photometer, *e.g.*, the DAWN-DSP photometer (Wyatt Technology Corp., Santa Barbara, CA), provides information for determination of the weight average molecular weight, determination of particle size, shape and particle-particle pair correlations.

### EXAMPLE 5: MOLECULAR WEIGHT DETERMINATION (DEGREE OF POLYMERIZATION) BY SUCROSE GRADIENT SEDIMENTATION

Linked assembly units of different lengths sediment at different rates in a sucrose gradient in zonal ultracentrifugation. The quantitative relationship between the degree of polymerization and sedimentation in Svedberg units is then calculated. This method is useful for characterizing the efficiency of self-assembly in general, as well as the process of staged assembly at each step of addition of a new assembly unit.

### EXAMPLE 6: MORPHOLOGY AND LENGTH OF RODS BY ELECTRON MICROSCOPY

After sucrose gradient fractionation and SDS-PAGE analysis, the partially purified fractions containing rods are apparent. Samples of the appropriate fractions are placed on EM grids and stained or shadowed to look for large structures using electron microscopy in order to determine their morphology.

## Claims

1. A method for staged assembly of a nanostructure comprising:
(a) contacting a nanostructure intermediate comprising at least one unbound joining element with an assembly unit comprising a plurality of different joining elements, wherein:
(i) none of the joining elements of said plurality of different joining elements can interact with itself or with another joining element of said plurality, and
(ii) a single joining element of said plurality and a single unbound joining element of the nanostructure intermediate are complementary joining element,
whereby the assembly unit is non-covalently bound to the nanostructure intermediate to form a new nanostructure intermediate for use in subsequent cycles;
(b) removing unbound assembly units; and
(c) repeating steps (a) and (b) for a sufficient number of cycles to form a nanostructure,
wherein the complementary joining elements in at least one cycle comprise pilin proteins or a binding derivative or binding fragment thereof.

2. The method of claim 1, wherein the nanostructure intermediate comprises a surface-bound initiator assembly unit.

3. The method of claim 1 or 2, comprising the additional step of:
(d) capping the nanostructure with at least one capping unit.

4. The method of any of claims 1 to 3, wherein a first assembly unit used in at least one cycle comprises at least one structural element covalently linked to a first joining element comprising pilin proteins or a binding derivative or binding fragment thereof.

5. The method of claim 4, wherein the structural element is covalently linked to the first joining element and to a second joining element.

6. The method of claim 5, wherein the second joining element comprises pilin proteins or a binding derivative or binding fragment thereof.

7. The method of claim 4, wherein the first assembly unit comprises a first structural element that is bound to a second structural element to form a stable complex.

8. The method of claim 4, wherein the assembly unit further comprises a functional element.

9. The method of any of claims 1 to 3, wherein a first assembly unit used in at least one cycle comprises a functional element and a joining element comprising pilin proteins or a binding derivative or binding fragment thereof.

10. The method of claim 8 or 9, wherein the functional element comprises a photoactive molecule, photonic nanoparticle, inorganic ion, inorganic nanoparticle, magnetic ion, magnetic nanoparticle, electronic nanoparticle, metallic nanoparticle, metal oxide nanoparticle, gold nanoparticle, gold-coated nanoparticle, carbon nanotube, nanocrystal, nanowire, quantum dot, peptide, protein, protein domain, enzyme, hapten, antigen, biotin, digoxygenin, lectin, toxin, radioactive label, fluorophore, chromophore, or chemiluminescent molecule.

11. The method of any of claims 1 to 10, further comprising the step of post-assembly conversion of specific non-covalent interactions of complementary joining elements to covalent linkages. whereby the linkages are stabilized.

12. The method of any of claims 1 to 11, wherein the assembly unit comprises a plurality of sub-assembly units that bind to each other to form a stable complex.

13. The method of any of claims 1 to 12, wherein the joining element in at least one cycle is a pilin protein and is selected from the group consisting of SEQ ID NOS: 1-10.

14. The method of any of claims 1 to 12, wherein the joining element in at least one cycle is a hybrid pilin protein.

15. The method of claim 15, wherein the hybrid pilin protein comprises an N-terminal extension sequence selected from the group consisting of SEQ ID NOS: 1, 3, 5, 7 and 9.

16. The method of claim 14 or 15, wherein the hybrid pilin protein comprises a pilin protein body sequence selected from the group consisting of SEQ ID NOS: 2, 4, 6, 8 and 10.

17. The method of claim 14 or 15, wherein the hybrid pilin protein comprises the pilin amino terminal extension of a first pilin protein and the pilin protein body of a second pilin protein and lacks the pilin protein body of the first pilin protein and the pilin amino terminal extension of the second pilin protein, wherein the amino terminal extension of the first pilin protein does not bind to the pilin protein body of the second pilin protein.

18. A nanostructure formed from a plurality of species of assembly units comprising a plurality of different joining elements, said assembly units including at least one species of assembly unit wherein at a first joining element comprises a pilin protein or binding derivative or binding fragment thereof.

19. The nanostructure of claim 18, wherein the first joining element comprises a pilin protein selected from the group consisting of SEQ ID NOS: 1-10

20. The nanostructure of claim 18, wherein the pilin protein is a hybrid pilin protein.

21. The nanostructure of claim 20, wherein the hybrid pilin protein comprises an N-terminal extension sequence selected from the group consisting of SEQ ID NOS: 1, 3, 5, 7 and 9.

22. The nanostructure of claim 20 or 21, wherein the hybrid pilin protein comprises a pilin protein body sequence selected from the group consisting of SEQ ID NOS: 2, 4, 6, 8 and 10.

23. The nanostructure of claim 20 or 21, wherein the hybrid pilin protein comprises the pilin amino terminal extension of a first pilin protein and the pilin protein body of a second pilin protein and lacks the pilin protein body of the first pilin protein and the pilin amino terminal extension of the second pilin protein, wherein the amino terminal extension of the first pilin protein does not bind to the pilin protein body of the second pilin protein.

24. The nanostructure of claim 18, made in accordance with any of claims 1 to 17.

25. A hybrid pilin protein, wherein the hybrid pilin protein comprises the pilin amino terminal extension of a first pilin protein and the pilin protein body of a second pilin protein, said pilin protein body including the carboxy terminus of the second pilin protein, and lacks the pilin protein body of the first pilin protein and the pilin amino terminal extension of the second pilin protein, wherein the amino terminal extension of the first pilin protein does not bind to the pilin protein body of the second pilin protein.

26. The hybrid pilin protein of claim 25, further comprising a functional element selected from the group consisting of photoactive molecule, photonic nanoparticle, inorganic ion, inorganic nanoparticle, magnetic ion, magnetic nanoparticle, electronic nanoparticle, metallic nanoparticle, metal oxide nanoparticle, gold nanoparticle, gold-coated nanoparticle, carbon nanotube, nanocrystal, nanowire, quantum dot, peptide, protein, protein domain, enzyme, hapten, antigen, biotin, digoxygenin, lectin, toxin, radioactive label, fluorophore, chromophore, and chemiluminescent molecule.

27. The hybrid pilin protein of claim 25 or 26, wherein the hybrid pilin protein comprises an N-terminal extension sequence selected from the group consisting of SEQ ID NOS: 1, 3, 5, 7 and 9.

28. The hybrid pilin protein of any of claims 25 to 27, wherein the hybrid pilin protein comprises a pilin protein body sequence selected from the group consisting of SEQ ID NOS: 2, 4, 6, 8 and 10.

## Patentansprüche

1. Ein Verfahren für das schrittweise Zusammenbauen einer Nanostruktur, welches Verfahren die folgenden Schritte aufweist:
(a) ein Nanostruktur-Zwischenprodukt mit einer Baueinheit in Berührung bringen, welches Zwischenprodukt mindestens ein ungebundenes Verbindungselement und welche Baueinheit eine Mehrzahl von voneinander verschiedenen Verbindungselementen aufweist,
wobei:
(i) keines der Verbindungselemente der genannten Mehrzahl von voneinander verschiedenen Verbindungselementen fähig ist, mit sich selbst oder mit einem anderen dieser Verbindungselemente zu reagieren, und
(ii) ein einzelnes Verbindungselement der Mehrzahl und ein einzelnes ungebundenes Verbindungselement des Nanostruktur-Zwischenproduktes komplementäre Verbindungselemente sind,
so dass die Baueinheit nicht-covalent an das Nanostruktur-Zwischenprodukt gebunden wird, um **dadurch** ein neues Nanostruktur-Zwischenprodukt für die Verwendung in nachfolgenden Zyklen zu bilden;
(b) ungebundene Baueinheiten entfernen; und
(c) - Schritte (a) und (b) für eine genügende Anzahl von Zyklen zur Bildung der Nanostruktur wiederholen,
wobei die komplementären Verbindungselemente in mindestens einem Zyklus Pilin-Proteine oder ein bindendes Derivat oder Fragment davon aufweisen.

2. Das Verfahren gemäss Anspruch 1, wobei das Nanostruktur-Zwischenprodukt eine an eine Oberfläche gebunene Initiator-Baueinheit aufweist.

3. Das Verfahren gemäss Anspruch 1 oder 2 mit einem zusätzlichen Schritt:
(d) Nanostruktur mit mindestens einer Abschlusseinheit abschliessen.

4. Das Verfahren gemäss irgendeinem der Ansprüche 1 bis 3, wobei eine in mindestens einem Zyklus verwendete erste Baueinheit mindestens ein Strukturelement aufweist, das covalent mit einem ersten Verbindungselement verbunden ist und das Pilin-Proteine oder ein bindendes Derivat oder Fragment davon aufweist.

5. Das Verfahren gemäss Anspruch 4, wobei das Strukturelement covalent an das erste Verbindungselement und an ein zweites Verbindungselement gebunden ist.

6. Das Verfahren gemäss Anspruch 5, wobei das zweite Verbindungselement Pilin-Proteine oder ein bindendes Derivat oder Fragment davon aufweist.

7. Das Verfahren gemäss Anspruch 4, wobei die erste Baueinheit ein erstes Strukturelement aufweist, welches mit einem zweiten Strukturelement verbunden ist und damit einen stabilen Komplex bildet.

8. Das Verfahren gemäss Anspruch 4, wobei die Baueinheit ferner ein Funktionselement aufweist.

9. Das Verfahren gemäss irgendeinem der Ansprüche 1 bis 3, wobei eine erste in mindestens einem Zyklus verwendete Baueinheit ein Funktionselement und ein Verbindungselement aufweist, die beide Pilin-Proteine oder ein bindendes Derivat oder Fragment davon aufweisen.

10. Das Verfahren gemäss Anspruch 8 oder 9, wobei das Funktionselement ein lichtempfindliches Molekül, Photon-Nanopartikel, anorganisches Ion, anorganisches Nanopartikel, magnetisches Ion, magnetisches Nanopartikel, elektronisches Nanopartikel, metallisches Nanopartikel, Metalloxid-Nanopartikel, Gold-Nanopartikel, goldbeschichtetes Nanopartikel, Kohlenstoff-Nanoröhre, Nanokristall, Nanodraht, Quantenfleck, Peptid, Protein, Proteindomäne, Enzym, Hapten, Antigen, Biotin, Digoxigenin, Lektin, Toxin, radioaktives Label, Fluorophor, Chromophor, oder ein Chemolumineszenz-Molekül aufweist.

11. Das Verfahren gemäss irgendeinem der Ansprüche 1 bis 10, wobei es einen weiteren Schritt aufweist, in dem nach dem Zusammenbauen spezifische nicht-covalente Verbindungen zwischen komplementären Verbindungselementen in covalente Bindungen gewandelt und **dadurch** stabilisiert werden.

12. Das Verfahren gemäss irgendeinem der Ansprüche 1 bis 11, wobei die Baueinheit eine Mehrzahl von Untereinheiten aufweist, welche miteinander verbunden sind und **dadurch** einen stabilen Komplex bilden.

13. Das Verfahren gemäss irgendeinem der Ansprüche 1 bis 12, wobei das Verbindungselement in mindestens einem Zyklus ein Pilin-Protein gemäss einer der SEQ ID Nummern 1 - 10 ist.

14. Das Verfahren gemäss irgendeinem der Ansprüche 1 bis 12, wobei das Verbindungselement in mindestens einem Zyklus ein Hybrid-Pilin-Protein ist.

15. Das Verfahren gemäss Anspruch 14, wobei das Hybrid-Pilin-Protein eine N-terminale Verlängerungssequenz gemäss einer der SEQ ID Nummern 1, 3, 5, 7 oder 9 aufweist.

16. Das Verfahren gemäss Anspruch 14 oder 15, wobei das Hybrid-Pilin-Protein eine Pilin-Protein-Körpersequenz gemäss einer der SEQ ID Nummern 2, 4, 6, 8 oder 10 aufweist.

17. Das Verfahren gemäss den Ansprüchen 14 oder 15, wobei das Hybrid-Pilin-Protein die aminoterminale Verlängerung eines ersten Hybrid-Pilins und den Proteinkörper eines zweiten Pilin-Proteins aufweist und den Proteinkörper des ersten Pilin-Proteins und die aminoterminale Verlängerung des zweiten Pilin-Proteins nicht aufweist, wobei die aminoterminale Verlängerung des ersten Pilin-Proteins sich nicht mit dem Proteinkörper des zweiten Pilin-Proteins verbindet.

18. Eine Nanostruktur gebildet aus einer Vielzahl von Arten von Baueinheiten, die je eine Mehrzahl von unterschiedlichen Verbindungselementen aufweisen, wobei die Baueinheiten mindestens einer der Arten als erstes Verbindungselement ein Pilin-Protein oder ein bindendes Derivat oder Fragment davon aufweisen.

19. Die Nanostruktur gemäss Anspruch 18, wobei das erste Verbindungselement ein Pilin-Protein gemäss einer der SEQ ID Nummern 1 - 10 aufweist.

20. Die Nanostruktur gemäss Anspruch 18, wobei das Pilin-Protein ein Hybrid-Pilin-Protein ist.

21. Die Nanostruktur gemäss Anspruch 20, wobei das Hybrid-Pilin-Protein eine N-terminale Verlängerungssequenz gemäss einer der SEQ ID Nummern 1, 3, 5, 7 oder 9 aufweist.

22. Die Nanostruktur gemäss Anspruch 20 oder 21, wobei das Hybrid-Pilin-Protein eine N-terminale Verlängerungssequenz gemäss einer der SEQ ID Nummern 2, 4, 6, 8 oder 10 aufweist.

23. Die Nanostruktur gemäss Anspruch 20 oder 21, wobei das Hybrid-Pilin Protein die aminoterminale Verlängerung eines ersten Pilin-Proteins und den Proteinkörper eines zweiten Pilin-Proteins aufweist und den Pilin-Proteinkörper des ersten Pilin-Proteins und die aminoterminale Verlängerung des zweiten Pilin-Proteins nicht aufweist, wobei die aminoterminale Verlängerung des ersten Pilin-Proteins sich nicht mit dem Proteinkörper des zweiten Pilin-Proteins verbindet.

24. Die Nanostruktur gemäss Anspruch 18, hergestellt mit dem Verfahren nach irgendeinem der Ansprüche 1 bis 17.

25. Ein Hybrid-Pilin-Protein, wobei das Hybrid-Pilin-Protein die aminoterminale Verlängerung eines ersten Pilin-Proteins und den Proteinkörper eines zweiten Pilin-Proteins aufweist, welcher Pilin-Proteinkörper den Carboxyterminus des zweiten Pilin-Proteins aufweist, wobei das Hybrid-Pilin-Protein den Proteinkörper des ersten Pilin-Proteins und die aminoterminale Verlängerung des zweiten Pilin-Proteins nicht aufweist, und wobei die aminoterminale Verlängerung des ersten Pilin-Proteins sich nicht mit dem Proteinkörper des zweiten Pilin-Proteins verbindet.

26. Das Hybrid-Pilin-Protein gemäss Anspruch 25, welches ferner als Funktionselement ein lichtempfindliches Molekül, Photon-Nanopartikel, anorganischem Ion, anorganischem Nanopartikel, magnetischem Ion, magnetischem Nanopartikel, elektronischem Nanopartikel, metallisches Nanopartikel, Metalloxid-Nanopartikel, Gold-Nanopartikel, goldbeschichtetes Nanopartikel, Kohlenstoff-Nanoröhre, Nanokristall, Nanodraht, Quantenfleck, Peptid, Protein, Proteindomäne, Enzym, Hapten, Antigen, Biotin, Digoxigenin, Lektin, Toxin, radioaktives Label, Fluorophor, Chromophor, oder ein Chemolumineszenz-Molekül aufweist.

27. Das Hybrid-Pilin-Protein gemäss Anspruch 25 oder 26, wobei das Hybrid- Protein eine N-terminale Verlängerungssequenz gemäss einer der SEQ ID Nummern 1, 3, 5, 7 oder 9 aufweist.

28. Das Hybrid-Pilin-Protein gemäss irgendeinem der Ansprüche 25 bis 27, wobei das Hybrid-Protein eine N-terminale Verlängerungssequenz gemäss einer der SEQ ID Nummern 2, 4, 6, 8 oder 10 aufweist.

## Revendications

1. Procédé d'assemblage par étapes d'une nanostructure, qui comprend les étapes qui consistent à :
(a) mettre un intermédiaire de nanostructure, qui comprend au moins un élément jointif non lié en contact avec une unité qui comprend plusieurs éléments jointifs différents, de telle sorte que :
(i) aucun desdits plusieurs éléments jointifs différents ne peut interagir avec lui-même ou avec un autre des plusieurs éléments jointifs et
(ii) un seul desdits plusieurs éléments jointifs et un seul élément jointif non lié de l'intermédiaire de nanostructure sont des éléments jointifs complémentaire,
l'unité d'assemblage étant reliée de manière non covalente à l'intermédiaire de nanostructure de manière à former un nouvel intermédiaire de nanostructure destiné à être utilisé dans des cycles ultérieurs,
(b) retirer les unités d'assemblage non liées et
(c) répéter les étapes (a) et (b) un nombre suffisamment de cycles de manière à former une nanostructure,
les éléments de jointifs complémentaires d'au moins un cycle comprenant des protéines de piline un_ dérivé _de liaison de celles-ci ou un fragment de liaison de celles-ci.

2. Procédé selon la revendication 1, dans lequel l'intermédiaire de nanostructure comprend une unité d'assemblage d'initiateur lié à une surface.

3. Procédé selon les revendications 1 ou 2, qui comprend l'étape supplémentaire qui consiste à:
(d) terminer la nanostructure par au moins une unité de terminaison.

4. Procédé selon l'une quelconque des revendications 1 à 3, dans lequel une première unité d'assemblage utilisée dans au moins un cycle comprend au moins un élément structurel lié par covalence à un premier élément jointif qui comprend des protéines de piline, un dérivé de liaison de celles-ci ou un fragment de liaison de celles-ci.

5. Procédé selon la revendication 4, dans lequel l'élément structurel est lié par covalence au premier élément jointif et à un deuxième élément jointif.

6. Procédé selon la revendication 5, dans lequel le deuxième élément jointif comprend des protéines de piline, un dérivé de liaison de celles-ci ou un fragment de liaison de celles-ci.

7. Procédé selon la revendication 4, dans lequel la première unité d'assemblage comprend un premier élément structurel lié à un deuxième élément structurel de manière à former un complexe stable.

8. Procédé selon la revendication 4, dans lequel l'unité d'assemblage comprend en outre un élément fonctionnel.

9. Procédé selon l'une quelconque des revendications 1 à 3, dans lequel une première unité d'assemblage utilisée dans au moins un cycle comprend un élément fonctionnel et un élément jointif qui contiennent des protéines de piline, un dérivé de liaison de celles-ci ou un fragment de liaison de celles-ci.

10. Procédé selon les revendications 8 ou 9, dans lequel l'élément fonctionnel peut être une molécule photoactive, une nanoparticule photonique, un ion minéral, une nanoparticule minérale, un ion magnétique, une nanoparticule magnétique, une nanoparticule électronique, une nanoparticule métallique, une nanoparticule d'oxyde métallique, une nanoparticule d'or, une nanoparticule revêtue d'or, un nanotube de carbone, un nanocristal, un nanofil, un point quantique, un peptide, une protéine, un domaine de protéine, une enzyme, un haptène, un antigène, une biotine, une digoxygénine, une lectine, une toxine, un marqueur radioactif, un fluorophore, un chromophore ou une molécule chimioluminescente.

11. Procédé selon l'une quelconque des revendications 1 à 10, qui comprend en outre après l'assemblage l'étape de conversion d'interactions non covalentes spécifiques d'éléments jointifs complémentaires en des liaisons covalentes, ce qui stabilise les liaisons.

12. Procédé selon l'une quelconque des revendications 1 à 11, dans lequel l'unité d'assemblage comprend plusieurs sous-unités d'assemblage qui se lient les unes aux autres pour former un complexe stable.

13. Procédé selon l'une quelconque des revendications 1 à 12, dans lequel l'élément jointif d'au moins un cycle est une protéine de piline et est sélectionné dans le groupe constitué des SEQ ID NO : 1 à 10.

14. Procédé selon l'une quelconque des revendications 1 à 12, dans lequel l'élément jointif d'au moins un cycle est une protéine de piline hybride.

15. Procédé selon la revendication 15, dans lequel la protéine de piline hybride comprend une séquence de prolongement de la terminaison N sélectionnée dans l'ensemble constitué des SEQ ID NO : 1, 3, 5, 7 ou 9.

16. Procédé selon les revendications 14 ou 15, dans lequel la protéine de piline hybride comporte une séquence de corps de protéine de piline sélectionnée dans l'ensemble constitué des SEQ ID NO : 2, 4, 6, 8 ou 10.

17. Procédé selon les revendications 14 ou 15, dans lequel la protéine de piline hybride comporte la prolongation de la terminaison amino d'une première protéine de piline et le corps d'une deuxième protéine de piline et est dépourvue du corps de la première protéine de piline et du prolongement de la terminaison amino de la deuxième protéine de piline, le prolongement de la terminaison amino de la première protéine de piline ne se liant pas au corps de la deuxième protéine de piline.

18. Nanostructure formée de plusieurs sortes d'unités d'assemblage qui comportent plusieurs éléments jointifs différents, lesdites unités d'assemblage comportant au moins une sorte d'unité d'assemblage dans laquelle un premier élément jointif comprend une protéine de piline, un dérivé de liaison de celle-ci ou un fragment de liaison de celle-ci.

19. Nanostructure selon la revendication 18, dans laquelle le premier élément jointif comprend une protéine de piline sélectionnée dans l'ensemble constitué des SEQ ID NO : 1 à 10.

20. Nanostructure selon la revendication 18, dans laquelle la protéine de piline est une protéine de piline hybride.

21. Nanostructure selon la revendication 20, dans laquelle la protéine de piline hybride comprend une séquence de prolongement de la terminaison N sélectionnée dans l'ensemble constitué des SEQ ID NO : 1, 3, 5, 7 et 9.

22. Nanostructure selon les revendications 20 ou 21, dans laquelle la protéine de piline hybride comprend une séquence de corps de protéine de piline sélectionnée dans l'ensemble constitué des SEQ ID NO : 2, 4, 6, 8 et 10.

23. Nanostructure selon les revendications 20 ou 21, dans laquelle la protéine de piline hybride comprend la prolongation de la terminaison amino d'une première protéine de piline et le corps d'une deuxième protéine de piline et est dépourvue du corps de la première protéine de piline et du prolongement de la terminaison amino de la deuxième protéine de piline, le prolongement de la terminaison amino de la première protéine de piline ne se liant pas au corps de la deuxième protéine de piline.

24. Nanostructure selon la revendication 18, réalisée selon l'une quelconque des revendications 1 à 17.

25. Protéine de piline hybride, qui comprend la prolongation de la terminaison amino d'une première protéine de piline et le corps d'une deuxième protéine de piline, ledit corps de protéine de piline comprenant la terminaison carboxy de la deuxième protéine de piline, et est dépourvue du corps de la première protéine de piline et du prolongement de la terminaison amino de la deuxième protéine de piline, le prolongement de la terminaison amino de la première protéine de piline ne se liant pas au corps de la deuxième protéine de piline.

26. Protéine de piline hybride selon la revendication 25, qui comprend en outre un élément fonctionnel sélectionné dans l'ensemble constitué des molécules photoactives, des nanoparticules photoniques, des ions minéraux, des nanoparticules minérales, des ions magnétiques, des nanoparticules magnétiques, des nanoparticules électroniques, des nanoparticules métalliques, des nanoparticules d'oxyde métallique, des nanoparticules d'or, des nanoparticules revêtues d'or, des nanotubes de carbone, des nanocristaux, des nanofils, des points quantiques, des peptides, des protéines, des domaines de protéine, des enzymes, des haptènes, des antigènes, des biotines, des digoxygénines, des lectines, des toxines, des marqueurs radioactifs, des fluorophores, des chromophores et des molécules chimioluminescentes.

27. Protéine de piline hybride selon les revendications 25 ou 26, qui comprend une séquence du prolongement de la terminaison N sélectionnée dans l'ensemble constitué des SEQ ID NO : 1, 3, 5, 7 et 9.

28. Protéine de piline hybride selon l'une quelconque des revendications 25 à 27, qui comprend une séquence de corps de protéine de piline sélectionnée dans l'ensemble constitué des SEQ ID NO : 2, 4, 6, 8 et 10.
